# EUROPEAN PATENT APPLICATION

(11) **EP 4 674 464 A1**
(43) Date of publication of application: **07.01.2026**
(21) Application number: 24853863.9
(22) Date of filing: 15.08.2024
(51) Int. Cl.: A61M 60/122, A61M 60/178

(54) **VENTRICULAR ASSIST SYSTEM, AND FLUSHING METHOD, CONSUMABLE AND DEVICE FOR MEDICAL CATHETER PUMP SYSTEM**

(30) Priority: 16.08.2023 CN 202311034302
(71) Applicant: Magassist Co., Ltd., Suzhou, Jiangsu 215163 (CN)
(72) Inventor: ZHANG, Jialiang, Suzhou, Jiangsu 215163 (CN); ZHAO, Ruixiong, Suzhou, Jiangsu 215163 (CN); GAO, Yong, Suzhou, Jiangsu 215163 (CN)
(74) Representative: Winter, Brandl - Partnerschaft mbB
(86) International application number: PCT/CN2024/112401
(87) International publication number: WO 2025/036457

(57) **Abstract**

The present application discloses a ventricular assist system, a flushing method, a consumable material, a control apparatus, and a control device for a medical catheter pump system, and belongs to the technical field of medical devices. In the ventricular assist system, the first three-way valve includes a first valve port, a second valve port, and a third valve port, wherein the first valve port is connected to a first flushing path driven by the flushing pump assembly, and the second valve port is connected to a first flushing interface of the medical catheter pump; the second three-way valve includes a fourth valve port, a fifth valve port, and a sixth valve port, wherein the fourth valve port is connected to a second flushing path driven by the flushing pump assembly, and the fifth valve port is connected to a second flushing interface of the medical catheter pump.

## Description

This application claims priority to Chinese Patent Application No. 2023110343026 filed to the China National Intellectual Property Administration on August 16, 2023 and entitled "Ventricular Assist System, Flushing Method , Consumable Material, and Apparatus for Medical Catheter Pump System", the application of which is hereby incorporated by reference in its entirety.

### Technical Field

Embodiments of the present application relate to the technical field of medical devices, and in particular, to a ventricular assist system, a flushing method, a consumable material, and an apparatus for a medical catheter pump system.

### Background

A medical catheter pump is a medical apparatus configured to assist the heart in providing blood circulation power. During working, a catheter of the medical catheter pump is delivered to a left ventricle via a femoral artery route, an inflow port is located at an outflow tract from the left ventricle, and an outflow port is located inside in an aorta; and an axial flow pump can pump blood out from the inflow port at a left ventricle end during operation, and then returns to the aorta through the outflow port at an aorta end, thereby assisting the heart.

It is necessary to empty air in the medical catheter pump first when the medical catheter pump is used. In a relevant art, a flushing pump is controlled to continuously prime the medical catheter pump with a flushing liquid through a flushing pipeline, and the flushing liquid squeezes the air, thereby eliminating air bubbles in the medical catheter pump.

However, a pipeline for flushing a catheter is relatively long, so by performing overall flushing on the flushing pipeline and the medical catheter pump, it is difficult to thoroughly eliminate the air in the overall catheter pump system, and air bubbles remain easily.

### Summary

The present application provides a ventricular assist system, a flushing method, a consumable material, and an apparatus for a medical catheter pump system. Different flushing positions in the ventricular assist system can be separately flushed or primed by switching a conduction state of a first three-way valve and a second three-way valve. The technical solution is as follows.

According to an aspect of the present application, a ventricular assist system is provided. The ventricular assist system includes: a flushing pump assembly, a first three-way valve, a second three-way valve, and a medical catheter pump, wherein the first three-way valve includes a first valve port, a second valve port, and a third valve port, the first valve port is connected to a first flushing path driven by the flushing pump assembly, and the second valve port is connected to a first flushing interface of the medical catheter pump; and the second three-way valve includes a fourth valve port, a fifth valve port, and a sixth valve port, the fourth valve port is connected to a second flushing path driven by the flushing pump assembly, and the fifth valve port is connected to a second flushing interface of the medical catheter pump.

In an embodiment, the first three-way valve and the second three-way valve have different conduction states when flushing or priming for different flushing positions are different, the flushing positions include the first flushing path and the second flushing path, or the flushing positions include an inner cavity of the medical catheter pump.

In an embodiment, in a first priming stage, the second valve port and the third valve port of the first three-way valve are conducted, the fifth valve port and the sixth valve port of the second three-way valve are conducted, the sixth valve port of the second three-way valve or the third valve port of the first three-way valve is connected to a flushing assembly, where the first priming stage includes a stage of priming the inner cavity of the medical catheter pump with the flushing liquid by using the flushing assembly.

In an embodiment, the flushing assembly includes an injection pump, an injector, a needle tube, and a pressurized liquid bag.

In an embodiment, in a second priming stage, the first valve port and the third valve port of the first three-way valve are conducted, and the fourth valve port and the sixth valve port of the second three-way valve are conducted, where the second priming stage includes a stage of priming the first flushing path and the second flushing path with the flushing liquid by using the flushing pump assembly.

In an embodiment, in a priming stage, any two valve ports of the first three-way valve are conducted, the fifth valve port and the sixth valve port of the second three-way valve are conducted, the sixth valve port of the second three-way valve or the third valve port of the first three-way valve is connected to a flushing assembly, where the priming stage includes a stage of priming the inner cavity of the medical catheter pump with the flushing liquid by using the flushing assembly.

In an embodiment, in a priming stage, the second valve port and the third valve port of the first three-way valve are conducted, the fifth valve port and the sixth valve port of the second three-way valve are conducted, and the sixth valve port of the second three-way valve or the third valve port of the first three-way valve is connected to the flushing assembly, where the priming stage includes a stage of priming the first flushing path and the second flushing path with the flushing liquid by using the flushing pump assembly, and a stage of priming the inner cavity of the medical catheter pump with the flushing liquid by using the flushing assembly.

In an embodiment, in an overall flushing stage, the first valve port and the second valve port of the first three-way valve are conducted, and the fourth valve port and the fifth valve port of the second three-way valve are conducted, where the overall flushing stage includes a stage of flushing a circulating flushing loop formed by the first flushing path, the second flushing path, and the inner cavity of the medical catheter pump.

In an embodiment, the flushing assembly includes a rapid flushing assembly, and an instantaneous flow rate of the flushing liquid provided by the rapid flushing assembly is greater than an instantaneous flow rate of the flushing liquid provided by the flushing pump assembly.

In an embodiment, the rapid flushing assembly includes a pressurizing apparatus; and an instantaneous flow rate of the flushing liquid provided by the pressurizing apparatus is greater than an instantaneous flow rate of the flushing liquid provided by the flushing pump assembly.

In an embodiment, the flushing pump assembly includes a flushing pump and a circulating pump. The flushing pump is configured to deliver the flushing liquid into the inner cavity of the medical catheter pump through the second flushing path in the overall flushing stage. The circulating pump is configured to control the flushing liquid to cyclically flush the inner cavity of the medical catheter pump in the overall flushing stage.

In an embodiment, the flushing pump is disposed at a common part of the first flushing path and the second flushing path located at a starting position, and the circulating pump is disposed on a path on the second flushing path that is not shared with the first flushing path.

In an embodiment, the system further includes a control device. The control device is configured to control a pumping flow rate corresponding to the flushing pump assembly.

According to another aspect of the present application, a flushing method for a medical catheter pump system is provided. The medical catheter pump system includes the medical catheter pump system includes a medical catheter pump and a flushing pipeline; a first flushing interface of the medical catheter pump is connected to a first flushing path driven by a flushing pump assembly through an in-valve path between a second valve port and a first valve port of a first three-way valve; a second flushing interface of the medical catheter pump is connected to a second flushing path driven by the flushing pump assembly through an in-valve path between a fifth valve port and a fourth valve port of a second three-way valve; the flushing pipeline forms the first flushing path and the second flushing path.

The method includes: in a priming stage, priming the first flushing path and the second flushing path with a flushing liquid by using the flushing pump assembly, and priming an inner cavity of the medical catheter pump with the flushing liquid by using a flushing assembly connected to a sixth valve port of the second three-way valve or a third valve port of the first three-way valve; and in an overall flushing stage, flushing a circulating flushing loop formed by the first flushing path, the second flushing path, and the inner cavity of the medical catheter pump by using the flushing pump assembly.

In an embodiment, the first three-way valve and the second three-way valve have different conduction states when flushing or priming is performed on different flushing positions, the flushing positions include the first flushing path and the second flushing path, or the flushing positions include an inner cavity of the medical catheter pump.

In an embodiment, the in a priming stage, the first flushing path and the second flushing path are primed with the flushing liquid by using the flushing pump assembly, and the inner cavity of the medical catheter pump is primed with the flushing liquid by using the flushing assembly connected to the sixth valve port of the second three-way valve, including that: in a first priming stage, the inner cavity of the medical catheter pump is primed with the flushing liquid by using the flushing assembly connected to the sixth valve port of the second three-way valve or the third valve port of the first three-way valve; and in a second priming stage, the first flushing path and the second flushing path are primed with the flushing liquid by using the flushing pump assembly.

In an embodiment, the method further includes: in the first priming stage, the second valve port and the third valve port of the first three-way valve are controlled to communicate with each other, and the fifth valve port and the sixth valve port of the second three-way valve are controlled to communicate with each other.

In an embodiment, the method further includes: in the second priming stage, the first valve port and the third valve port of the first three-way valve are controlled to communicate with each other, and the fourth valve port and the sixth valve port of the second three-way valve are controlled to communicate with each other.

In an embodiment, in the priming stage, the first flushing path and the second flushing path are primed with the flushing liquid by using the flushing pump assembly, and the inner cavity of the medical catheter pump is primed with the flushing liquid by using the flushing assembly connected to the sixth valve port of the second three-way valve, including that: in the priming stage, the inner cavity of the medical catheter pump is primed with the flushing liquid by using the flushing assembly connected to the sixth valve port of the second three-way valve or the third valve port of the first three-way valve; and simultaneously, the first flushing path and the second flushing path are primed with the flushing liquid by using the flushing pump assembly.

In an embodiment, the method further includes: in the priming stage, any two valve ports of the first three-way valve are controlled to communicate with each other, and the fifth valve port and the sixth valve port of the second three-way valve are controlled to communicate with each other.

In an embodiment, the method further includes: in the priming stage, the second valve port and the third valve port of the first three-way valve are controlled to communicate with each other; and the fifth valve port and the sixth valve port of the second three-way valve are controlled to communicate with each other.

In an embodiment, the method further includes: in the priming stage, any two valve ports of the second three-way valve are controlled to communicate with each other, and the second valve port and the third valve port of the first three-way valve are controlled to communicate with each other.

In an embodiment, the flushing assembly includes a rapid flushing assembly, and the inner cavity of the medical catheter pump is primed with the flushing liquid by using the flushing assembly connected to the sixth valve port of the second three-way valve or the third valve port of the first three-way valve, including that: the inner cavity of the medical catheter pump is primed with the flushing liquid by using the rapid flushing assembly connected to the sixth valve port of the second three-way valve or the third valve port of the first three-way valve, and an instantaneous flow rate of the flushing liquid provided by the rapid flushing assembly is greater than an instantaneous flow rate of the flushing liquid provided by the flushing pump assembly.

In an embodiment, the flushing pump assembly includes a flushing pump and a circulating pump, the flushing pump is disposed at a common part of the first flushing path and the second flushing path located at a starting position, and the circulating pump is disposed on a path on the second flushing path that is not shared with the first flushing path, wherein, the priming the first flushing path and the second flushing path with a flushing liquid by using the flushing pump assembly includes: controlling a rotating speed of the flushing pump and a rotating speed of the circulating pump to enable a difference between a flushing liquid flow rate of a first sub-path and a flushing fluid flow rate of a second sub-path to be within a preset range, wherein the first sub-path includes a path on the first flushing path that is not shared with the second flushing path, and the second sub-path includes a path on the second flushing path that is not shared with the first flushing path; priming the first flushing path and the second flushing path with the flushing liquid by using the flushing pump and the circulating pump.

In an embodiment, the method further includes: in a transition stage before entering the overall flushing stage, controlling a pumping flow rate of the flushing pump assembly to be a second flow rate, wherein the second flow rate is less than a first flow rate, and the first flow rate is a pumping flow rate of the flushing pump assembly in the priming stage.

In an embodiment, in the transition stage, any two valve ports of the first three-way valve are controlled to communicate with each other, and the fifth valve port and the sixth valve port of the second three-way valve are controlled to communicate with each other.

In an embodiment, in the transition stage, the second valve port and the third valve port of the first three-way valve are controlled to communicate with each other; and the fifth valve port and the sixth valve port of the second three-way valve are controlled to communicate with each other.

In an embodiment, in the transition stage, any two valve ports of the second three-way valve are controlled to communicate with each other, and the second valve port and the three valve port of the first three-way valve are controlled to communicate with each other.

In an embodiment, the method further includes: in the transition stage, prompt information for switching to the overall flushing stage is displayed; and a confirm operation is received in response to the prompt information, and the overall flushing stage is entered.

In an embodiment, the method further includes: in the overall flushing stage, the first valve port and the second valve port of the first three-way valve are controlled to communicate with each other, and the fourth valve port and the fifth valve port of the second three-way valve are controlled to communicate with each other.

In an embodiment, the flushing pump assembly includes a flushing pump and a circulating pump, the flushing pump is disposed at a common part of the first flushing path and the second flushing path located at a starting position, and the circulating pump is disposed on a path on the second flushing path that is not shared with the first flushing path; the in an overall flushing stage, flushing a circulating flushing loop formed by the first flushing path, the second flushing path, and the inner cavity of the medical catheter pump by using the flushing pump assembly includes: in the overall flushing stage, controlling a rotating speed of the flushing pump based on a flushing liquid flow rate of a catheter of the medical catheter pump; and controlling a rotating speed of the circulating pump based on a flushing liquid flow rate of the circulating flushing loop formed by the first flushing path, the second flushing path, and the inner cavity of the medical catheter pump; and flushing the circulating flushing loop and the catheter of the medical catheter pump by using the flushing pump and the circulating pump.

According to another aspect of the present application, a flushing method for a medical catheter pump system is provided. The medical catheter pump system includes a medical catheter pump and a flushing pipeline; a first flushing interface of the medical catheter pump is connected to a first flushing path driven by a flushing pump assembly through an in-valve path between a second valve port and a first valve port of a first three-way valve; a second flushing interface of the medical catheter pump is connected to a second flushing path driven by the flushing pump assembly through an in-valve path between a fifth valve port and a fourth valve port of a second three-way valve; the flushing pipeline forms the first flushing path and the second flushing path; and the method is performed by a control device corresponding to the flushing pump assembly, the control device is connected to the flushing pump assembly, and the method includes: in a priming stage, controlling the flushing pump assembly to prime the first flushing path and the second flushing path with a flushing liquid; and in an overall flushing stage, controlling the flushing pump assembly to flush a circulating flushing loop formed by the first flushing path, the second flushing path, and the inner cavity of the medical catheter pump.

In an embodiment, the first three-way valve and the second three-way valve have different conduction states when flushing or priming is performed on different flushing positions, the flushing positions include the first flushing path and the second flushing path, or the flushing positions include an inner cavity of the medical catheter pump.

In an embodiment, the flushing pump assembly includes a flushing pump and a circulating pump. The flushing pump is disposed at a common part of the first flushing path and the second flushing path located at a starting position, and the circulating pump is disposed on a path on the second flushing path that is not shared with the first flushing path; in the priming stage, the first flushing path and the second flushing path are primed with the flushing liquid by using the flushing pump assembly, including that: in the priming stage, a rotating speed of the flushing pump and a rotating speed of the circulating pump are controlled to enable a difference between a flushing liquid flow rate of a first sub-path and a flushing fluid flow rate of a second sub-path to be within a preset range, where the first sub-path includes a path on the first flushing path that is not shared with the second flushing path, and the second sub-path includes a path on the second flushing path that is not shared with the first flushing path; and the first flushing path and the second flushing path are primed with the flushing liquid by using the flushing pump and the circulating pump.

In an embodiment, the method further includes: in the transition stage before entering the overall flushing stage, a pumping flow rate of the flushing pump is controlled to be a second flow rate, the second flow rate is less than a first flow rate, and the first flow rate is a pumping flow rate of the flushing pump in the priming stage.

In an embodiment, the flushing pump assembly includes a flushing pump and a circulating pump. The flushing pump is disposed at a common part of the first flushing path and the second flushing path located at a starting position, and the circulating pump is disposed on a path on the second flushing path that is not shared with the first flushing path; in the overall flushing stage, the flushing pump assembly is controlled to flush the circulating flushing loop formed by the first flushing path, the second flushing path, and the inner cavity of the medical catheter pump, including that: in the overall flushing stage, a rotating speed of the flushing pump is controlled based on a flushing liquid flow rate of a catheter of the medical catheter pump; a rotating speed of the circulating pump is controlled based on a flushing liquid flow rate of the circulating flushing loop formed by the first flushing path, the second flushing path, and the inner cavity of the medical catheter pump; and the circulating flushing loop and the catheter of the medical catheter pump are flushed by using the flushing pump and the circulating pump.

According to another aspect of the present application, a combustible material for a medical catheter pump system is provided. The combustible material includes a first three-way valve and a second three-way valve; a first valve port of the first three-way valve is connected to a first flushing path driven by a flushing pump assembly, and a second valve port of the first three-way valve is connected to a first flushing interface of a medical catheter pump; and a fourth valve port of the second three-way valve is connected to a second flushing path driven by the flushing pump assembly, and a fifth valve port of the second three-way valve is connected to a second flushing interface of the medical catheter pump.

In an embodiment, the combustible material further includes a flushing pipeline, wherein the flushing pipeline forms the first flushing path and the second flushing path, the first three-way valve is disposed at a tail end of a pipe orifice corresponding to the first flushing path, and the second three-way valve is disposed at a tail end of a pipe orifice corresponding to the second flushing path.

In an embodiment, the combustible material further includes the medical catheter pump, wherein the medical catheter pump includes: a catheter; a drive shaft, rotatably penetrating through the catheter; a pump head, including: a pump housing connected to a distal end of the catheter, and an impeller accommodated in the pump housing, wherein the impeller is connected to a distal end of the drive shaft to be driven to rotate to pump blood; and a drive catheter handle, including a rotor capable of being driven by a motor, wherein a proximal end of the drive shaft is connected to the rotor, and an inner cavity for rotatably supporting the rotor in the drive catheter handle is formed in the drive catheter handle; the catheter is communicated with the inner cavity; and the drive catheter handle is provided with the first flushing interface and the second flushing interface that are communicated with the inner cavity, the first three-way valve is disposed at the first flushing interface, and the second three-way valve is disposed at the second flushing interface, wherein a part of a flushing liquid entering the inner cavity through the second flushing interface enters the catheter and is completely discharged to a human body at the pump head, and an other part is discharged from the first flushing interface after passing through the rotor.

According to another aspect of the present application, a control apparatus for a medical catheter pump system is provided. The medical catheter pump system includes a medical catheter pump and a flushing pipeline; a first flushing interface of the medical catheter pump is connected to a first flushing path driven by a flushing pump assembly through an in-valve path between a second valve port and a first valve port of a first three-way valve; a second flushing interface of the medical catheter pump is connected to a second flushing path driven by the flushing pump assembly through an in-valve path between a fifth valve port and a fourth valve port of a second three-way valve; the flushing pipeline forms the first flushing path and the second flushing path; and the apparatus includes: a control module, configured to: in a priming stage, prime the first flushing path and the second flushing path with a flushing liquid by using the flushing pump assembly, and prime an inner cavity of the medical catheter pump with the flushing liquid by using a flushing assembly connected to a sixth valve port of the second three-way valve.

The control module is further configured to: in an overall flushing stage, flush a circulating flushing loop formed by the first flushing path, the second flushing path, and the inner cavity of the medical catheter pump by using the flushing pump assembly.

In an embodiment, the first three-way valve and the second three-way valve have different conduction states when flushing or priming is performed on different flushing positions, the flushing positions include the first flushing path and the second flushing path, or the flushing positions include an inner cavity of the medical catheter pump.

According to another aspect of the present application, a control apparatus for a medical catheter pump system is provided. The medical catheter pump system includes a medical catheter pump and a flushing pipeline; a first flushing interface of the medical catheter pump is connected to a first flushing path driven by a flushing pump assembly through an in-valve path between a second valve port and a first valve port of a first three-way valve; a second flushing interface of the medical catheter pump is connected to a second flushing path driven by the flushing pump assembly through an in-valve path between a fifth valve port and a fourth valve port of a second three-way valve; the flushing pipeline forms the first flushing path and the second flushing path; and the control apparatus includes: a control module, configured to: in a priming stage, control the flushing pump assembly to prime the first flushing path and the second flushing path with a flushing liquid, wherein the control module is further configured to: in an overall flushing stage, control the flushing pump assembly to flush a circulating flushing loop formed by the first flushing path, the second flushing path, and the inner cavity of the medical catheter pump.

In an embodiment, the first three-way valve and the second three-way valve have different conduction states when flushing or priming is performed on different flushing positions, the flushing positions include the first flushing path and the second flushing path, or the flushing positions include an inner cavity of the medical catheter pump.

In an embodiment, the control apparatus further includes: a flushing pump assembly. The flushing pump assembly includes a flushing pump and a circulating pump, wherein the flushing pump is configured to deliver the flushing liquid into the inner cavity of the medical catheter pump through the second flushing path in the overall flushing stage, and the circulating pump is configured to control the flushing liquid to cyclically flush the inner cavity of the medical catheter pump in the overall flushing stage.

In an embodiment, the flushing pump is disposed at a common part of the first flushing path and the second flushing path located at a starting position, and the circulating pump is disposed on a path on the second flushing path that is not shared with the first flushing path.

According to another aspect of the present application, a computer storage medium is provided, having at least one computer program stored therein, where the at least one computer program is loaded and executed by a processor to implement the method for flushing the medical catheter pump system as described in the above aspect.

According to another aspect of the present application, a computer program product is provided. The above computer program product includes a computer program, and the computer program is stored in a computer storage medium. The computer program is read and executed by a processor of the computer device from the computer storage medium, so that the computer device performs the method for flushing the medical catheter pump system as described in the above aspect.

According to another aspect of the present application, a chip is provided. The chip includes a programmable logic circuit or a program, and a device installed with the chip is configured to implement the method for flushing the medical catheter pump system as described in the above aspect.

The technical solutions provided by the present application bring at least the following beneficial effects: the first three-way valve and the second three-way valve have different conduction states when flushing or priming is performed on different flushing positions. The flushing positions include, but are not limited to, the first flushing path, the second flushing path, and the inner cavity of the medical catheter pump. Different flushing positions in the ventricular assist system can be separately flushed or primed by switching the conduction state of the first three-way valve and the second three-way valve to implement priming or flushing for different flushing positions separately. Compared with priming or flushing the overall position at one time, the method for dividing the flushing positions to perform priming or flushing can enable each flushing position to be sufficiently flushed or primed, thereby exhausting air at different flushing positions more thoroughly, avoiding air bubbles remaining in some narrow gaps, and improving an exhaust effect of priming or flushing an overall system.

### Brief Description of the Drawings

To describe technical solutions in embodiments of the present application more clearly, accompanying drawings required for the description of the embodiments are briefly introduced below. Apparently, the accompanying drawings described below are only some embodiments of the present application, and those of ordinary skill in the art can further obtain other drawings based on these accompanying drawings without creative effort.
Fig. 1 is a schematic diagram of an application scenario of a medical catheter pump according to an exemplary embodiment of the present application.
Fig. 2 is a schematic diagram of a ventricular assist system according to an exemplary embodiment of the present application.
Fig. 3 is a schematic diagram of a ventricular assist system in a first priming stage according to an exemplary embodiment of the present application.
Fig. 4 is a schematic diagram of a ventricular assist system in a second priming stage according to an exemplary embodiment of the present application.
Fig. 5 is a schematic diagram of a ventricular assist system in an overall flushing stage according to an exemplary embodiment of the present application.
Fig. 6 is a flowchart of a method for flushing a medical catheter pump system according to an exemplary embodiment of the present application.
Fig. 7 is a flowchart of a method for flushing a medical catheter pump system in a case of priming in a staged manner according to an exemplary embodiment of the present application.
Fig. 8 is a flowchart of a method for flushing a medical catheter pump system in a case of priming simultaneously according to an exemplary embodiment of the present application.
Fig. 9 is a flowchart of a method for flushing a medical catheter pump system in an existence of a transition stage according to an exemplary embodiment of the present application.
Fig. 10 is a flowchart of a method for flushing a medical catheter pump system according to an exemplary embodiment of the present application.
Fig. 11 is a flowchart of a method for flushing a medical catheter pump system according to an exemplary embodiment of the present application.
Fig. 12 is a schematic diagram of a ventricular assist system according to another exemplary embodiment of the present application.
Fig. 13 is a schematic diagram of a ventricular assist system according to another exemplary embodiment of the present application.
Fig. 14 is a schematic diagram of a ventricular assist system according to another exemplary embodiment of the present application.
Fig. 15 is a block diagram of an apparatus for flushing a medical catheter pump according to an exemplary embodiment of the present application.
Fig. 16 is a block diagram of an apparatus for flushing a medical catheter pump according to an exemplary embodiment of the present application.

101, drive motor; 102, catheter; 103, pump head;
110, flushing pipeline; 120, catheter pump; 121, drive catheter handle; 122, catheter; 123, pump head; 130, control device; 131, console; 132, drive motor; 140, liquid bag; 170, connector;
1501, control module; 1502, display module; 1601, control module;
210, flushing pump assembly; 211, flushing pump; 212, circulating pump; 220, first three-way valve; 221, first valve port; 222, second valve port; 223, third valve port; 230, second three-way valve; 231, fourth valve port; 232, fifth valve port; 233, sixth valve port; 240, medical catheter pump; 241, drive catheter handle; 242, catheter; 243, pump head; 250, flushing liquid container; and 260, flushing assembly.

### Detailed Description of the Embodiments

To make objectives, technical solutions, and advantages of the present application clearer, the following further describes implementations of the present application in detail with reference to accompanying drawings. Exemplary embodiments are described in detail herein, and examples of the exemplary embodiments are shown in the accompanying drawings. When the following description involves the accompanying drawings, unless otherwise indicated, the same numerals in different accompanying drawings represent the same or similar members. Implementations described in the following exemplary embodiments do not represent all implementations consistent with the present application. On the contrary, they are only examples of apparatuses and methods that are described in the appended claims in detail and that are consistent with some aspects of the present application.

Terms used in the present application are merely used for the purpose of describing specific embodiments and are not intended to limit the present application. Singular forms such as "a", "the", and "this" used in the present application and the appended claims are intended to include plural forms, unless other meanings are clearly indicated in context. It is further to be understood that the term "and/or" used herein indicates and includes any or all possible combinations of one or more associated listed items.

It is to be understood that although terms such as "first", "second", and "third" are used in the present application to describe various types of information, the information is not limited to these terms. These terms are merely used to distinguish between information of the same type.

A ventricular assist system is an auxiliary circulation apparatus system that replaces a ventricle to work, including, but not limited to, an extracorporeal ventricular assist system, a transcatheter ventricular assist system, and an implantable ventricular assist system.

The transcatheter ventricular assist system includes a control host for the transcatheter ventricular assist system (subsequently referred to as a control device for short) and a catheter pump system for the transcatheter ventricular assist system (subsequently referred to as a medical catheter pump system), which is an interventional blood pump, and is suitable for providing temporary left ventricular circulation assistance during related surgeries.

The medical catheter pump refers to a catheter pump configured to assist the heart in providing blood circulation power in a medical scenario. Optionally, the medical catheter pump is a cardiac pump that intervenes in an organism. Optionally, part or all components in the medical catheter pump intervene an organism.

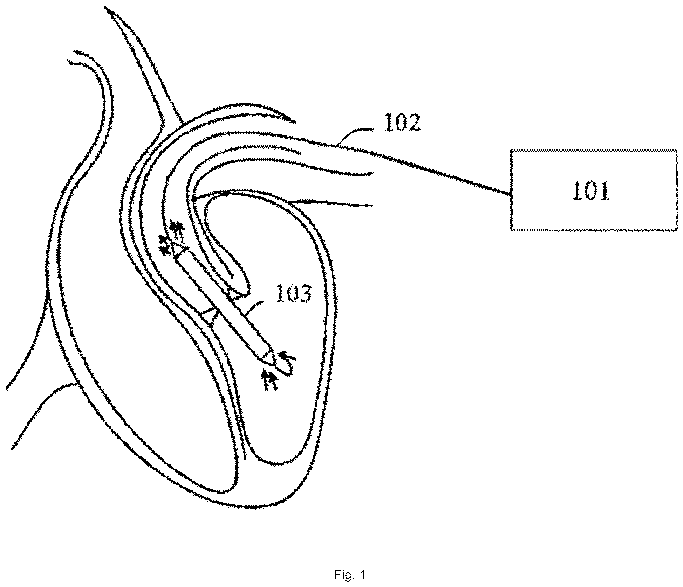

Fig. 1 is a schematic diagram of an application scenario of a medical catheter pump according to an embodiment of the present application. The medical catheter pump includes a drive catheter handle (not shown in the figures), a catheter 102, and a pump head 103. A drive motor 101 is coupled to a proximal end of the catheter 102 through the drive catheter handle, and the pump head 103 is connected to a distal end of the catheter 102.

The pump head 103 in the medical catheter pump can be percutaneously placed into the heart through a peripheral blood vessel along with the catheter 102, and the pump head 103 is placed between the left ventricle and the ascending aorta. A blood inlet of the pump head 103 is placed into the left ventricle, a blood outlet of the pump head 103 is placed into the ascending aorta, the drive motor is connected to an interventional pump to drive an impeller in the pump head to rotate, thereby pumping blood from the left ventricle into the ascending aorta, and implementing a ventricular assist function.

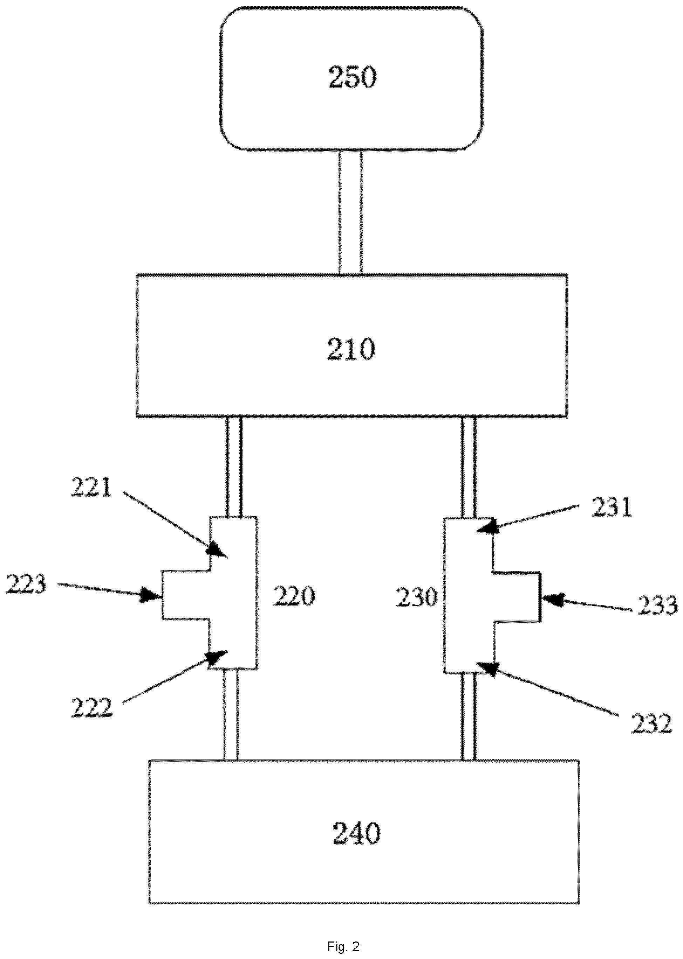

Fig. 2 is a schematic diagram of a ventricular assist system according to an exemplary embodiment of the present application. The ventricular assist system includes a flushing pump assembly 210, a first three-way valve 220, a second three-way valve 230, and a medical catheter pump 240.

A first valve port 221 of the first three-way valve 220 is connected to a first flushing path driven by a flushing pump assembly 210, a second valve port 222 of the first three-way valve 220 is connected to the medical catheter pump 240, the second valve port 222 of the first three-way valve 220 is connected to a first flushing interface (not shown in the figure) of the medical catheter pump 240, and the first three-way valve 220 further has a third valve port 223. The ventricular assist system can further include a flushing liquid container 250. The first flushing path includes a path formed by connecting the first three-way valve 220 and the flushing liquid container 250 by using a pipeline.

A fourth valve port 231 of the second three-way valve 230 is connected to a second flushing path driven by the flushing pump assembly 210, a fifth valve port 232 of the second three-way valve 230 is connected to the medical catheter pump 240, the fifth valve port 232 of the second three-way valve 230 is connected to a second flushing interface (not shown in the figure) of the medical catheter pump 240, the second three-way valve 230 further has a sixth valve port 233. The second flushing path includes a path formed by connecting the second three-way valve 230 and the flushing liquid container 250 by using a pipeline.

A three-way valve refers to a valve body with three valve ports. The valve ports of the three-way valve are controlled to be conducted or closed to form an in-valve path. For example, the first valve port and the second valve port are conducted to form an in-valve path, the first valve port and the third valve port are conducted to form an in-valve path, the second valve port and the third valve port are conducted to form an in-valve path, and any two of the first valve port, the second valve port, and the third valve port are conducted to form an in-valve path.

The three-way valve is provided with a control structure. The control structure can control conduction state of any two valve ports of the three-way valve. The above control structure is a pure mechanical structure, or an electric control mechanical structure. The conduction state of the three-way valve can be manually adjusted, it can also be controlled by a control device.

The first three-way valve and the second three-way valve have different conduction states when flushing or priming for different flushing positions are different. The flushing positions include, but are not limited to, the first flushing path, the second flushing path, or the inner cavity of the medical catheter pump. Different positions in the ventricular assist system can be separately flushed or primed by switching the conduction state of the first three-way valve and the second three-way valve to implement priming or flushing for different flushing positions separately. Compared with priming or flushing the overall flushing position at one time, the method for dividing the flushing positions to perform priming or flushing can enable each flushing position to be sufficiently flushed or primed, thereby exhausting air at different positions more thoroughly, avoiding air bubbles remaining in some narrow gaps, and improving an exhaust effect of priming or flushing an overall system.

In an optional embodiment, the ventricular assist system further includes a control device. The control device is configured to control a pumping flow rate corresponding to the flushing pump assembly. The control device controls the flushing pump assembly, thereby more stably controlling a pumping flow rate for priming or flushing. **In** addition, the control device can further control the conduction states of the first three-way valve and the second three-way valve, so that a flushing liquid can perform flushing or priming on different positions under a pumping action of the flushing pump assembly.

Before the medical catheter pump percutaneously enters a human body to work, it is necessary to flush the medical catheter pump to exhaust air in the medical catheter pump. A flushing process includes a priming stage and an overall flushing stage. The priming stage is used for rapidly priming the flushing liquid, and the overall flushing stage is used for continuously flushing an overall flushing path.

In an embodiment, the priming stage includes a first priming stage and a second priming stage. The first priming stage and the second priming stage are used for priming different flushing positions with the flushing liquid. The first priming stage is a stage for performing priming on an inner cavity of the medical catheter pump, thereby exhausting air from the inner cavity of the medical catheter pump, and preventing air bubbles from remaining in small gaps in the inner cavity of the medical catheter pump. The second priming stage is a stage for performing priming on a flushing pipeline in the medical catheter pump, thereby exhausting air from the flushing pipeline.

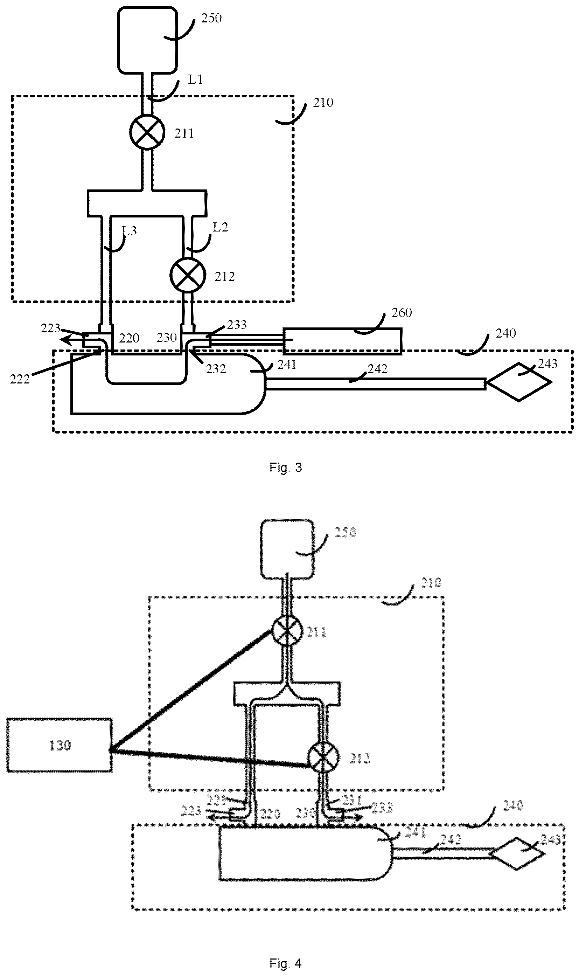

For the first priming stage, Fig. 3 is a schematic diagram of a ventricular assist system in a first priming stage according to an exemplary embodiment of the present application. The ventricular assist system includes a flushing pump assembly 210, a first three-way valve 220, a second three-way valve 230, and a medical catheter pump 240.

In the first priming stage, a second valve port 222 and a third valve port 223 of the first three-way valve 220 are conducted, a fifth valve port 232 and a sixth valve port 233 of the second three-way valve 230 are conducted, the sixth valve port 233 of the second three-way valve 230 is connected to a flushing assembly 260, or the third valve port 223 of the first three-way valve 220 is connected to a flushing assembly 260. In the present application, the first three-way valve refers to a three-way valve located on a left side in the figure, and the second three-way valve refers to a three-way valve located on a right side in the figure. In the present application, an example in which the sixth valve port 233 of the second three-way valve 230 is connected to the flushing assembly 260 is only used, and similarly, the flushing assembly 260 may be connected to the third valve port 223 of the first three-way valve 220. The flushing assembly is configured to provide a flushing liquid required for priming the inner cavity of the medical catheter pump. The first priming stage includes a stage of priming the inner cavity of the medical catheter pump with the flushing liquid by using the flushing assembly.

In the first priming stage, under a pumping action of the flushing assembly 260, the flushing liquid flows into the second three-way valve 230 through the sixth valve port 233 of the second three-way valve 230, and enters an inner cavity of a drive catheter handle 241 through the fifth valve port 232 of the second three-way valve 230. The flushing liquid enters the inner cavity to exhaust air in the inner cavity, enters the first three-way valve 220 through the second valve port 222 of the first three-way valve 220, and then is discharged through the third valve port 223 of the first three-way valve 220. Within a period of time, the flushing liquid is injected according to the above pathway, thereby completing priming of the flushing liquid in the inner cavity of the medical catheter pump, and exhausting the air in the inner cavity.

The first three-way valve and the second three-way valve are disposed between the flushing path and the medical catheter pump, so that the medical catheter pump can be connected to an additional flushing assembly through the three-way valves, and the inner cavity of the medical catheter pump is flushed by using the additional flushing assembly, thereby effectively exhausting the air in the inner cavity, and preventing air bubbles from remaining in small gaps of the inner cavity.

Optionally, the flushing assembly includes at least one of an injection pump, an injector, a needle tube, and a pressurized liquid bag.

Optionally, the flushing assembly 260 is a rapid flushing assembly, and an instantaneous flow rate of the flushing liquid provided by the rapid flushing assembly is greater than an instantaneous flow rate of the flushing liquid provided by the flushing pump assembly 210. Optionally, the rapid flushing assembly includes a pressurizing device. Optionally, the rapid flushing assembly is an injector. Specifically, the instantaneous flow rate provided by the rapid flushing assembly for the inner cavity of the medical catheter pump is greater than the instantaneous flow rate provided by the flushing pump assembly 210 for the inner cavity of the medical catheter pump.

When the inner cavity is flushed, a greater instantaneous flow rate is provided by using the rapid flushing assembly to rapidly inject the flushing liquid into the inner cavity, so that the air in the inner cavity is exhausted more rapidly, and residual air bubbles do not appear easily at a great instantaneous flow rate. Compared with a manner of performing priming by pumping the flushing liquid into the inner cavity by using the flushing pump assembly, and a better exhausting effect for performing priming on the inner cavity is achieved by providing the great instantaneous flow rate by using the rapid flushing assembly.

In an exemplary embodiment, the above medical catheter pump 240 includes:
a catheter 242, a drive shaft, a pump head 243, and a drive catheter handle 241.

The drive shaft (not shown in the figure) rotatably penetrates through the catheter 242.

The pump head 243 includes: a pump housing connected to a distal end of the catheter 242, and an impeller accommodated in the pump housing. The impeller is connected to a distal end of the drive shaft to be driven to rotate to pump blood.

The drive catheter handle 241 includes a rotor capable of being driven by a motor. A proximal end of the drive shaft is connected to the rotor. An inner cavity for rotatably supporting the rotor in the drive catheter handle is formed in the drive catheter handle. The catheter is communicated with the inner cavity. The drive catheter handle is provided with a first flushing interface and a second flushing interface that are communicated with the inner cavity. The first three-way valve 220 is disposed at the first flushing interface, and the second three-way valve 230 is disposed at the second flushing interface.

Part of a flushing liquid entering the inner cavity through the second flushing interface enters the catheter 242 and is completely discharged to a human body at the pump head 243, and the other part is discharged from the first flushing interface after passing through the rotor.

Specifically, the drive catheter handle 241 includes a coupling housing that is connected to the proximal end of the catheter 242 and that is detachably connected to a drive motor corresponding to the medical catheter pump 240. An inner cavity for rotatably supporting the rotor in the coupling housing is formed in the coupling housing. The coupling housing is provided with the first flushing interface and the second flushing interface that are communicated with the inner cavity.

Fig. 3 further illustrates that the flushing pump assembly 210 includes a flushing pump 211 and a circulating pump 212. Functions of the flushing pump 211 and the circulating pump 212 will be introduced in hereinafter. Optionally, the flushing pump 211 and a circulating pump 212 can pump a liquid by squeezing a pipeline, for example, a roller pump and a peristaltic pump. This is not limited in embodiments of this the present application. Optionally, the flushing pump and the peristaltic pump can have an inlet and an outlet. The flushing liquid enters the pump through the inlet, is pumped through a pumping mechanism (for example, an impeller), and is discharged from the outlet.

In an exemplary embodiment, the above ventricular assist system further includes a flushing pipeline. The flushing pipeline forms the above first flushing path and second flushing path.

In a possible implementation, the above flushing pipeline includes a supplementary pipeline L1 connected to a flushing liquid container 250. The supplementary pipeline L1 is communicated with a flushing liquid inlet pipe L2 and a flushing liquid outlet pipe L3 through a communication structure. The flushing liquid inlet pipe L2 is connected to the above second three-way valve, and is communicated with the second flushing interface through the above second three-way valve; and the flushing liquid outlet pipe L3 is connected to the above first three-way valve, and is communicated with the first flushing interface through the first three-way valve. The above flushing liquid inlet pipe L2, the inner cavity of the medical catheter pump, and the flushing liquid outlet pipe L3 form a circulating flushing loop. The flushing liquid cyclically flushes in the loop to cool the inner cavity of the medical catheter pump. Under this structure, the above supplementary pipeline L1 and the above flushing liquid outlet pipe L3 form the above first flushing path through the above communication structure, and the above supplementary pipeline L1 and the above flushing liquid inlet pipe L2 form the above second flushing path through the above communication structure. Optionally, the above supplementary pipeline L1, through the communication structure, the above flushing liquid inlet pipe L2, and the above flushing liquid inlet pipe L3 is integrally formed pipelines, or assembled and connected pipelines.

Optionally, the circulating pump is disposed on the circulating flushing loop to drive the flushing liquid to flow. For example, the circulating pump is disposed on the above flushing liquid inlet pipe L2 or flushing liquid outlet pipe L3, and pumps the flushing liquid in a manner of squeezing a pipeline. Optionally, the flushing pump is disposed on the above supplementary pipeline L1 to supplement the flushing liquid to the circulating flushing loop.

In another possible implementation, the above flushing pipeline can include a flushing liquid inlet pipe and a flushing liquid outlet pipe. The flushing liquid inlet pipe and the flushing liquid outlet pipe are separately connected to the flushing liquid container 250. The flushing liquid inlet pipe is connected to the above second three-way valve, and is communicated with the second flushing interface through the above second three-way valve. The flushing liquid inlet pipe is connected to the above first three-way valve, and is communicated with the first flushing interface through the first three-way valve. The above flushing liquid inlet pipe, the inner cavity of the medical catheter pump, and the flushing liquid outlet pipe can still form a circulating flushing loop.

Optionally, the circulating pump is disposed on the circulating flushing loop to drive the flushing liquid to flow. For example, the circulating pump is disposed on the above flushing liquid outlet pipe, and pumps a flushing liquid that flows out back to the flushing liquid container in a manner of squeezing a pipeline. Optionally, the flushing pump is disposed on the above flushing liquid inlet pipe to pump the flushing liquid to the inner cavity of the medical catheter pump.

For the second priming stage, Fig. 4 is a schematic diagram of a ventricular assist system in the second priming stage according to an exemplary embodiment of the present application. The ventricular assist system includes a flushing pump assembly 210, a first three-way valve 220, a second three-way valve 230, and a medical catheter pump 240.

In the second priming stage, a first valve port 221 and a third valve port 223 of the first three-way valve 220 are conducted, a fourth valve port 231 and a sixth valve port 233 of the second three-way valve 230 are conducted, where the second priming stage includes a stage of priming a first flushing path and a second flushing path with a flushing liquid by using the flushing pump assembly 210. The ventricular assist system further includes a flushing liquid container 250. The first flushing path includes a path formed by connecting the first three-way valve 220 and the flushing liquid container 250 by using a flushing pipeline. The second flushing path includes a path formed by connecting the second three-way valve 230 and the flushing liquid container 250 by using a flushing pipeline.

In the second priming stage, if the first flushing path and the second flushing path are respectively connected to the first three-way valve 220 and the second three-way valve 230, a flushing liquid in the flushing liquid container 250 is injected into each of the first flushing path and the second flushing path under a pumping action of the flushing pump assembly 210, and is discharged from each of the third valve port 223 of the first three-way valve 220 and the sixth valve port 233 of the second three-way valve 230, thereby priming the flushing liquid into the first flushing path and the second flushing path, and exhausting the air in the flushing path and the second flushing path. If the first flushing path and the second flushing path are not connected to the first three-way valve 220 and the second three-way valve 230, the flushing liquid is discharged from a pipe orifice of each of the first flushing path and the second flushing path.

Fig. 4 further illustrates that the flushing pump assembly 210 includes a flushing pump 211 and a circulating pump 212. The flushing pump 211 is disposed at a common part of the first flushing path and the second flushing path located at a starting position, and the circulating pump 212 is disposed on a path on the second flushing path that is not shared with the first flushing path. For the common part, refer to the supplementary pipeline L1 described above. For the path on the second flushing path that is not shared with the first flushing path, refer to the flushing liquid inlet pipe L2 described above. Details are not described herein again.

Fig. 4 further illustrates that the medical catheter pump 240 includes a drive catheter handle 241, a catheter 242, and a pump head 243.

Compared with a manner of flushing the flushing path and the inner cavity together (in this manner, the flushing liquid passes through the flushing path first and then enters the inner cavity, and the flushing liquid is pumped by the flushing pump assembly, so it is difficult to form a high flow rate, it is difficult to exhaust the air in the inner cavity, and air bubbles will remain in the inner cavity), in this embodiment of the present application, a length of a priming route for the inner cavity of the medical catheter pump is reduced, thereby avoiding an impact on an exhausting effect for priming the medical catheter pump by the flushing path, rapidly exhausting air bubbles in a flushing pipeline, and prevent small air bubbles from remaining in the flushing pipeline.

It is understood that, for the above first priming stage and the second priming stage, the first priming stage is performed first and then the second priming stage is performed, or the second priming stage is performed first and then the first priming stage is performed. The above distinction between the two priming stages is only to describe priming at different times.

In conclusion, priming is performed in two priming stages, and different flushing positions are primed with a flushing liquid separately. Compared with a manner of priming various flushing positions together, the flushing positions are primed with the flushing liquid one by one in a staged manner, thereby effectively improving priming exhaust effect of the various flushing positions, and preventing air bubbles from remaining gaps in the various flushing positions.

In an embodiment, the priming stage is not divided into a first priming stage and a second priming stage (the above first priming stage and second priming stage are performed simultaneously). The priming stage includes a stage of priming the first flushing path and the second flushing path with the flushing liquid by using the flushing pump assembly, and a stage of priming the inner cavity of the medical catheter pump with the flushing liquid by using the flushing assembly.

For the priming stage, a possible on-off state of a three-way valve is provided. Any two valve ports of the first three-way valve 220 are conducted, the fifth valve port 232 and the sixth valve port 233 of the second three-way valve 230 are conducted, the sixth valve port 233 of the second three-way valve 230 or the third valve port 223 of the first three-way valve 220 is connected to the flushing assembly 260. The flushing assembly 260 is configured to provide a flushing liquid required for priming the inner cavity of the medical catheter pump. The priming stage includes a stage of priming the first flushing path and the second flushing path with the flushing liquid by using the flushing pump assembly 210, and a stage of priming the inner cavity of the medical catheter pump 240 with the flushing liquid by using the flushing assembly 260.

In this on-off state, the first flushing path remains connected to the first three-way valve 220, and the second flushing path is disconnected from the second three-way valve 230. The flushing liquid in the flushing liquid container 250 is injected into each of the first flushing path and the second flushing path under a pumping action of the flushing pump assembly 210. The flushing liquid in the first flushing path is discharged from the third valve port 223 of the first three-way valve 220, and the flushing liquid in the second flushing path is discharged from a pipe orifice at a tail end of the second flushing path, thereby implementing priming of the first flushing path and the second flushing path, and exhausting the air in the first flushing path and the second flushing path.

An inner cavity priming stage synchronized with the above pipeline priming process is as follows: under a pumping action of the flushing assembly 260, the flushing liquid flows into the second three-way valve 230 through the sixth valve port 233 of the second three-way valve 230, and enters an inner cavity of a drive catheter handle 241 through the fifth valve port 232 of the second three-way valve 230. The flushing liquid enters the inner cavity to exhaust air in the inner cavity, enters the first three-way valve 220 through the second valve port 222 of the first three-way valve 220, and then is discharged through the third valve port 223 of the first three-way valve 220.

For the priming stage, another possible on-off state of a three-way valve is provided. the second valve port 222 and the third valve port 223 of the first three-way valve 220 are conducted, the fifth valve port 232 and the sixth valve port 233 of the second three-way valve 230 are conducted, and the sixth valve port 233 of the second three-way valve 230 or the third valve port 223 of the first three-way valve 220 is connected to the flushing assembly. The flushing assembly is configured to provide a flushing liquid required for priming the inner cavity of the medical catheter pump.

In this on-off state, the first flushing path is disconnected from the first three-way valve 220, and the second flushing path is disconnected from the second three-way valve 230. The flushing liquid in the flushing liquid container 250 is injected into each of the first flushing path and the second flushing path under a pumping action of the flushing pump assembly 210, and is discharged from a pipe orifice at a tail end of each of the first flushing path and the second flushing path, thereby implementing priming of the first flushing path and the second flushing path, and exhausting the air in the first flushing path and the second flushing path.

An inner cavity priming process synchronized with the above pipeline priming process is as follows: under a pumping action of the flushing assembly 260, the flushing liquid flows into the second three-way valve 230 through the sixth valve port 233 of the second three-way valve 230, and enters an inner cavity of a drive catheter handle 241 through the fifth valve port 232 of the second three-way valve 230. The flushing liquid enters the inner cavity to exhaust air in the inner cavity, enters the first three-way valve 220 through the second valve port 222 of the first three-way valve 220, and then is discharged through the third valve port 223 of the first three-way valve 220.

Optionally, the flushing assembly is a rapid flushing assembly. The inner cavity of the medical catheter pump is primed with the flushing liquid by using the rapid flushing assembly connected to the sixth valve port of the second three-way valve or the third valve port of the first three-way valve, and an instantaneous flow rate of the flushing liquid provided by the rapid flushing assembly is greater than an instantaneous flow rate of the flushing liquid provided by the flushing pump assembly. Optionally, the rapid flushing assembly is an injector. Specifically, the instantaneous flow rate provided by the rapid flushing assembly for the inner cavity of the medical catheter pump is greater than the instantaneous flow rate provided by the flushing pump assembly for the inner cavity of the medical catheter pump.

In conclusion, a method for simultaneously priming is provided. Simultaneously priming can shorten time, and meet a time requirement in clinical practice. The first flushing path, the second flushing path, and the inner cavity of the medical catheter pump are simultaneously primed with the flushing liquid by using the flushing pump assembly and the flushing assembly. Compared with priming the first flushing path, the second flushing path, and the inner cavity of the medical catheter pump with the flushing liquid by using the flushing pump assembly, the flushing assembly is introduced to prime the flushing liquid at the same time, the flushing liquid provided by the flushing assembly is mainly configured to prime the inner cavity of the medical catheter pump, and an additional flushing liquid flow rate is introduced to accelerate an overall flushing process.

Compared with a manner of performing overall flushing on the flushing path and the inner cavity in a relevant art (in this manner, the flushing liquid passes through the flushing path first and then enters the inner cavity, and the flushing liquid is pumped by the flushing pump assembly, so it is difficult to form a high flow rate, it is difficult to exhaust the air in the inner cavity, and air bubbles will remain in the inner cavity), in this embodiment of the present application, an additional flushing assembly is introduced to prime the inner cavity of the medical catheter pump, so that the flushing liquid can be rapidly injected into the inner cavity, the air in the inner cavity is exhausted more rapidly, and at an instantaneous high flow rate, residual bubbles did not readily appear. Compared with a manner of performing priming by pumping the flushing liquid into the inner cavity by using the flushing pump assembly, and a better exhausting effect for performing priming on the inner cavity is achieved by providing the great instantaneous flow rate by using the rapid flushing assembly.

The on-off state of the three-way valve in the priming stage has been described above. Next, the on-off state of the three-way valve in the overall flushing stage will be introduced. The overall flushing stage includes a stage of flushing a circulating flushing loop formed by the first flushing path, the second flushing path, and the inner cavity of the medical catheter pump 240 by using the flushing pump assembly 210.

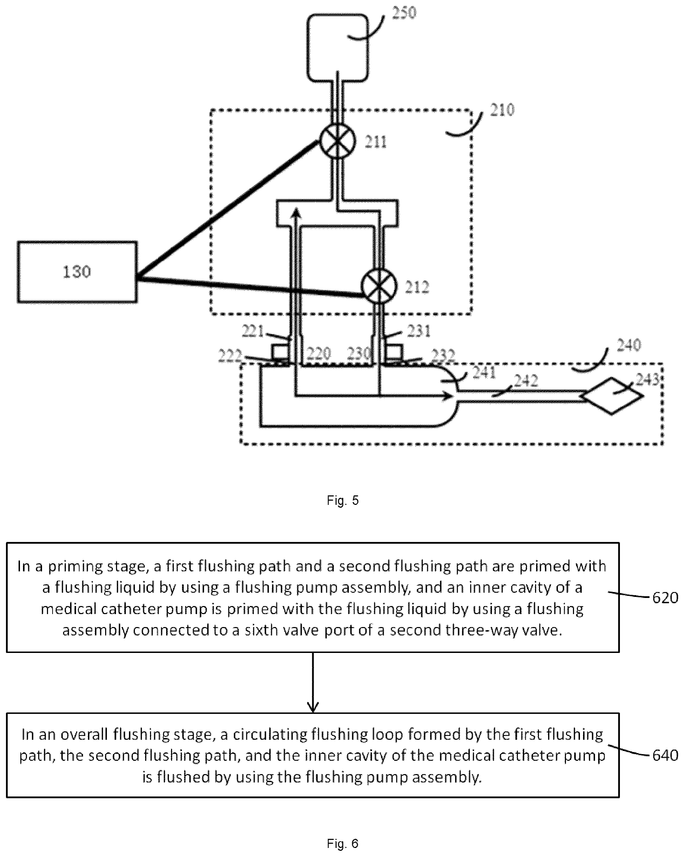

Fig. 5 is a schematic diagram of a ventricular assist system in an overall flushing stage according to an exemplary embodiment of the present application. The ventricular assist system includes a flushing pump assembly 210, a first three-way valve 220, a second three-way valve 230, and a medical catheter pump 240.

For the overall flushing stage, a first valve port 221 and a second valve port 222 of the first three-way valve 220 are conducted, and a fourth valve port 231 and a fifth valve port 232 of the second three-way valve 230 are conducted.

Fig. 5 further illustrates that the flushing pump assembly 210 includes a flushing pump 211 and a circulating pump 212. The flushing pump 211 is configured to deliver a flushing liquid into an inner cavity of the medical catheter pump 240 through the second flushing path in the overall flushing stage, and the circulating pump 212 is configured to control the flushing liquid to cyclically flush the inner cavity of the medical catheter pump 240 in the overall flushing stage.

The flushing pump 211 is disposed at a common part of the first flushing path and the second flushing path located at a starting position, for example, the above supplementary pipeline L1; and the circulating pump 212 is disposed on a path on the second flushing path that is not shared with the first flushing path, for example, the above flushing liquid inlet pipe L2. Fig. 5 further illustrates that the medical catheter pump 240 includes a drive catheter handle 241, a catheter 242, and a pump head 243.

Fig. 5 illustrates an overall flushing stage. Under a pumping action of the flushing pump assembly 210, the flushing liquid in the flushing liquid container 250 is injected into the second flushing path, then into the inner cavity of the drive catheter handle 241, then cyclically flows to the first flushing path; and the flushing liquid passing through the first flushing path flows into the second flushing path again.

Moreover, part of the flushing liquid flowing into the inner cavity of the drive catheter handle 241 will flow into the catheter 242 of the medical catheter pump 240, and is completely discharged at the pump head 243.

Fig. 6 is a flushing method for a medical catheter pump system according to an exemplary embodiment of the present application. The medical catheter pump system includes a medical catheter pump and a flushing pipeline. The method includes the following steps.

At step 620, in a priming stage, a first flushing path and a second flushing path are primed with a flushing liquid by using a flushing pump assembly, and an inner cavity of a medical catheter pump is primed with the flushing liquid by using a flushing assembly connected to a sixth valve port of a second three-way valve or a third valve port of a first three-way valve. An example in which the flushing assembly is connected to the third valve port of the first three-way valve is only used for describing.

With reference to Fig. 2, in the present application, a first flushing interface (not shown in the figure) of a medical catheter pump 240 is connected to the first flushing path driven by a flushing pump assembly 210 through an in-valve path between a second valve port 222 and a first valve port 221 of a first three-way valve 220. The first three-way valve 220 further has a third valve port 223. Optionally, the ventricular assist system further includes a flushing liquid container 250. The first flushing path includes a path formed by connecting the first three-way valve 220 and the flushing liquid container 250 by using a flushing pipeline.

A second flushing interface (not shown in the figure) of the medical catheter pump 240 is connected to the second flushing path driven by the flushing pump assembly 210 through an in-valve path between a fifth valve port 232 and a fourth valve port 231 of the second three-way valve 230. The second three-way valve 230 further has a sixth valve port 233. Optionally, the ventricular assist system further includes a flushing liquid container 250. The second flushing path includes a path formed by connecting the second three-way valve 230 and the flushing liquid container 250 by using a flushing pipeline.

At least one flushing pipeline forms the first flushing path and the second flushing path, as described in the embodiment in Fig. 3. Details are not described herein again. The flushing liquid refers to a liquid for squeezing and exhausting air in the present application.

When the flushing liquid is primed in a priming stage, the flushing liquid in the first flushing path will flow out from the flushing liquid container through the first flushing path or the first three-way valve. The flushing liquid in the second flushing path will flow out from the flushing liquid container through the second flushing path or the second three-way valve.

When the flushing liquid is primed in the priming stage, the flushing liquid will flow from the second three-way valve to the first three-way valve through the inner cavity of the medical catheter pump.

Optionally, the medical catheter pump includes a drive catheter handle (that is, the inner cavity), a catheter, and a pump head.

At step 640, in an overall flushing stage, a circulating flushing loop formed by the first flushing path, the second flushing path, and the inner cavity of the medical catheter pump is flushed by using the flushing pump assembly.

Optionally, in the overall flushing stage, a control device controls the flushing pump assembly to flush the circulating flushing loop formed by the first flushing path, the second flushing path, and the inner cavity of the medical catheter pump.

In the overall flushing stage, the first valve port and the second valve port of the first three-way valve are controlled to communicate with each other, and the fourth valve port and the fifth valve port of the second three-way valve are controlled to communicate with each other. The first three-way valve and the second three-way valve are mechanical valves or electric control valves.

In the overall flushing stage, the circulating flushing loop formed by the first flushing path, the second flushing path, and the inner cavity of the medical catheter pump, and the pump head of the medical catheter pump are flushed by using the flushing pump assembly.

In conclusion, the first three-way valve and the second three-way valve have different conduction states when flushing or priming is performed on different flushing positions. The flushing positions include, but are not limited to, the first flushing path, the second flushing path, or the inner cavity of the medical catheter pump. Different positions in the ventricular assist system can be separately flushed or primed by switching the conduction state of the first three-way valve and the second three-way valve to implement priming or flushing for different flushing positions separately. Compared with priming or flushing the overall flushing position at one time, the method for dividing the flushing positions to perform priming or flushing can enable each flushing position to be sufficiently flushed or primed, thereby exhausting air at different positions more thoroughly, avoiding air bubbles remaining in some narrow gaps, and improving an exhaust effect of priming or flushing an overall system.

Moreover, the first three-way valve and the second three-way valve are disposed between the flushing path and the medical catheter pump, so that the medical catheter pump can be connected to an additional flushing assembly through the three-way valves, and the medical catheter pump is flushed by using the additional flushing assembly, thereby exhausting the air in the inner cavity of the medical catheter pump, and preventing air bubbles from remaining in small gaps in the inner cavity of the medical catheter pump.

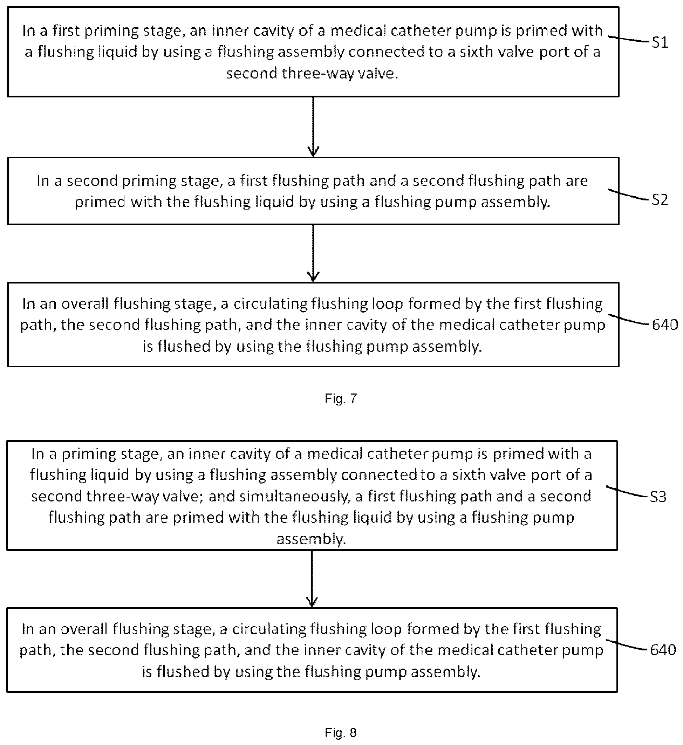

Based on an optional embodiment shown in Fig. 6, a priming stage in step 620 includes a first priming stage and a second priming stage. Fig. 7 is a flowchart of a flushing method for a medical catheter pump system according to an exemplary embodiment of the present application. Step 620 is replaced with the following S1 and S2.

At S1, in a first priming stage, an inner cavity of a medical catheter pump is primed with a flushing liquid by using a flushing assembly connected to a sixth valve port of a second three-way valve or a third valve port of a first three-way valve.

In the first priming stage, the second valve port and the third valve port of the first three-way valve are controlled to communicate with each other, and the fifth valve port and the sixth valve port of the second three-way valve are controlled to communicate with each other. With reference to Fig. 3, Fig. 3 illustrates that a second valve port 222 and a third valve port 223 of the first three-way valve 220 are conducted, and a fifth valve port 232 and a sixth valve port 233 of the second three-way valve 230 are conducted in this case. The sixth valve port 233 of the second three-way valve 230 is connected to a flushing assembly 260.

Optionally, the flushing assembly includes a rapid flushing assembly. In the first priming stage, the inner cavity of the medical catheter pump is primed with the flushing liquid by using the rapid flushing assembly connected to the sixth valve port of the second three-way valve or the third valve port of the first three-way valve, wherein an instantaneous flow rate of the flushing liquid provided by the rapid flushing assembly is greater than an instantaneous flow rate of the flushing liquid provided by the flushing pump assembly. Optionally, the rapid flushing assembly is an injector. Specifically, the instantaneous flow rate provided by the rapid flushing assembly for the inner cavity of the medical catheter pump is greater than the instantaneous flow rate provided by the flushing pump assembly for the inner cavity of the medical catheter pump.

At S2, in a second priming stage, a first flushing path and a second flushing path are primed with the flushing liquid by using a flushing pump assembly.

Optionally, in the second priming stage, a control device controls the flushing pump assembly to prime the first flushing path and the second flushing path with the flushing liquid.

In the second priming stage, the first valve port and the third valve port of the first three-way valve are controlled to communicate with each other, and the fourth valve port and the sixth valve port of the second three-way valve are controlled to communicate with each other. With reference to Fig. 4, Fig. 4 illustrates that a first valve port 221 and a third valve port 223 of the first three-way valve 220 are conducted, and a fourth valve port 231 and a sixth valve port 233 of the second three-way valve 230 are conducted in this case.

Optionally, the flushing pump assembly includes a flushing pump and a circulating pump. The flushing pump is disposed at a common part of the first flushing path and the second flushing path located at a starting position, and the circulating pump is disposed on a path on the second flushing path that is not shared with the first flushing path.

In an embodiment, in the second priming stage, a rotating speed of the flushing pump and a rotating speed of the circulating pump are controlled to enable a difference between a flushing liquid flow rate of a first sub-path and a flushing fluid flow rate of a second sub-path to be within a preset range. Optionally, the flushing liquid flow rate of the first sub-path is the same as or similar to the flushing fluid flow rate of the second sub-path, where the first sub-path includes a path on the first flushing path that is not shared with the second flushing path, and the second sub-path includes a path on the second flushing path that is not shared with the first flushing path; and the first flushing path and the second flushing path are primed with the flushing liquid by using the flushing pump and the circulating pump.

In an embodiment, the rotating speed of the flushing pump and the rotating speed of the circulating pump are controlled to enable the first flushing path and the second flushing path to complete priming the flushing liquid as soon as possible. In clinical practice, a priming time of the flushing liquid is as short as possible.

For example, under the structure of Fig. 4, the rotating speed of the flushing pump is twice the rotating speed of the circulating pump. In this way, the flushing flow rate of the first flushing path and the second flushing path are remained relatively consistent, so that the first flushing path and the second flushing path can be primed synchronously and rapidly.

In conclusion, priming is performed in two priming stages, and different flushing positions are primed with a flushing liquid separately. Compared with a manner of priming various flushing positions together, the flushing positions are primed with the flushing liquid one by one in a staged manner, thereby effectively improving priming exhaust effect of the various flushing positions, and preventing air bubbles from remaining gaps in the various flushing positions.

In an optional embodiment shown in Fig. 6, a priming stage in step 620 is not distinguished into a first priming stage and a second priming stage. Fig. 8 is a flowchart of a flushing method for a medical catheter pump system according to an exemplary embodiment of the present application. Step 620 is replaced with the following S3.

At S3, in a priming stage, an inner cavity of a medical catheter pump is primed with a flushing liquid by using a flushing assembly connected to a sixth valve port of a second three-way valve or a third valve port of a first three-way valve; and simultaneously, a first flushing path and a second flushing path are primed with the flushing liquid by using a flushing pump assembly.

Optionally, a control device controls the flushing pump assembly to prime the first flushing path and the second flushing path with the flushing liquid.

In an embodiment, in the priming stage, any two valve ports of the first three-way valve are controlled to communicate with each other, and the fifth valve port and the sixth valve port of the second three-way valve are controlled to communicate with each other. (In this case, the sixth valve port of the second three-way valve is connected to a flushing assembly).

In this on-off state, the first flushing path remains connected to the first three-way valve 220, and the second flushing path is disconnected from the second three-way valve 230. The flushing liquid in the flushing liquid container 250 is injected into each of the first flushing path and the second flushing path under a pumping action of the flushing pump assembly 210. The flushing liquid in the first flushing path is discharged from the third valve port 223 of the first three-way valve 220, and the flushing liquid in the second flushing path is discharged from a pipe orifice at a tail end of the second flushing path, thereby implementing priming of the first flushing path and the second flushing path, and exhausting the air in the first flushing path and the second flushing path.

An inner cavity priming stage synchronized with the above pipeline priming process is as follows: under a pumping action of the flushing assembly 260, the flushing liquid flows into the second three-way valve 230 through the sixth valve port 233 of the second three-way valve 230, and enters an inner cavity of a drive catheter handle 241 through the fifth valve port 232 of the second three-way valve 230. The flushing liquid enters the inner cavity to exhaust air in the inner cavity, enters the first three-way valve 220 through the second valve port 222 of the first three-way valve 220, and then is discharged through the third valve port 223 of the first three-way valve 220.

Alternatively, in an embodiment, in the priming stage, the second valve port and the third valve port of the first three-way valve are controlled to communicate with each other; and the fifth valve port and the sixth valve port of the second three-way valve are controlled to communicate with each other. (In this case, the sixth valve port of the second three-way valve is connected to a flushing assembly, or the third valve port of the first three-way valve is connected to the flushing assembly).

In this on-off state, the first flushing path is disconnected from the first three-way valve 220, and the second flushing path is disconnected from the second three-way valve 230. The flushing liquid in the flushing liquid container 250 is injected into each of the first flushing path and the second flushing path under a pumping action of the flushing pump assembly 210, and is discharged from a pipe orifice at a tail end of each of the first flushing path and the second flushing path, thereby implementing priming of the first flushing path and the second flushing path, and exhausting the air in the first flushing path and the second flushing path.

An inner cavity priming stage synchronized with the above pipeline priming process is as follows: under a pumping action of the flushing assembly 260, the flushing liquid flows into the second three-way valve 230 through the sixth valve port 233 of the second three-way valve 230, and enters an inner cavity of a drive catheter handle 241 through the fifth valve port 232 of the second three-way valve 230. The flushing liquid enters the inner cavity to exhaust air in the inner cavity, enters the first three-way valve 220 through the second valve port 222 of the first three-way valve 220, and then is discharged through the third valve port 223 of the first three-way valve 220.

Alternatively, in an embodiment, in the priming stage, any two valve ports of the second three-way valve are controlled to communicate with each other, and the second valve port and the third valve port of the first three-way valve are controlled to communicate with each other. (In this case, the third valve port of the first three-way valve is connected to a flushing assembly).

In this on-off state, the second flushing path remains connected to the second three-way valve 230, and the first flushing path is disconnected from the first three-way valve 220. The flushing liquid in the flushing liquid container 250 is injected into each of the first flushing path and the second flushing path under a pumping action of the flushing pump assembly 210. The flushing liquid in the second flushing path is discharged from the sixth valve port 233 of the second three-way valve 230, and the flushing liquid in the first flushing path is discharged from a pipe orifice at a tail end of the first flushing path, thereby implementing priming of the first flushing path and the second flushing path, and exhausting the air in the first flushing path and the second flushing path.

An inner cavity priming stage synchronized with the above pipeline priming process is as follows: under a pumping action of the flushing assembly 260, the flushing liquid flows into the second three-way valve 220 through the third valve port 223 of the first three-way valve 220, and enters an inner cavity of a drive catheter handle 241 through the second valve port 222 of the first three-way valve 220. The flushing liquid enters the inner cavity to exhaust air in the inner cavity, enters the second three-way valve 230 through the fifth valve port 232 of the second three-way valve 230, and then is discharged through the sixth valve port 233 of the second three-way valve 230.

In the priming stage, the sixth valve port of the second three-way valve or the third valve port of the first three-way valve is connected to a flushing assembly.

Optionally, the flushing assembly includes a rapid flushing assembly. The inner cavity of the medical catheter pump is primed with the flushing liquid by using the rapid flushing assembly connected to the sixth valve port of the second three-way valve or the third valve port of the first three-way valve, and an instantaneous flow rate of the flushing liquid provided by the rapid flushing assembly is greater than an instantaneous flow rate of the flushing liquid provided by the flushing pump assembly. Optionally, the rapid flushing assembly is an injector. Specifically, the instantaneous flow rate provided by the rapid flushing assembly for the inner cavity of the medical catheter pump is greater than the instantaneous flow rate provided by the flushing pump assembly for the inner cavity of the medical catheter pump.

Optionally, the flushing pump assembly includes a flushing pump and a circulating pump, the flushing pump is disposed at a common part of the first flushing path and the second flushing path located at a starting position, and the circulating pump is disposed on a path on the second flushing path that is not shared with the first flushing path.

In an embodiment, a rotating speed of the flushing pump and a rotating speed of the circulating pump are controlled to enable a difference between a flushing liquid flow rate of a first sub-path and a flushing fluid flow rate of a second sub-path to be within a preset range. Optionally, the flushing liquid flow rate of the first sub-path is the same as or similar to the flushing fluid flow rate of the second sub-path, where the first sub-path includes a path on the first flushing path that is not shared with the second flushing path, and the second sub-path includes a path on the second flushing path that is not shared with the first flushing path; and the first flushing path and the second flushing path are primed with the flushing liquid by using the flushing pump and the circulating pump.

In an embodiment, the rotating speed of the flushing pump and the rotating speed of the circulating pump are controlled to enable the first flushing path and the second flushing path to complete priming the flushing liquid as soon as possible. In clinical practice, a priming time of the flushing liquid is as short as possible.

For example, the rotating speed of the flushing pump is twice the rotating speed of the circulating pump. In this way, the flushing flow rate of the first flushing path and the second flushing path are remained relatively consistent, so that the first flushing path and the second flushing path can be primed synchronously and rapidly.

In conclusion, a method for simultaneously priming is provided. Simultaneously priming can shorten time, and meet a time requirement in clinical practice. A first flushing pipeline, a second flushing pipeline, and the inner cavity of the medical catheter pump are simultaneously primed with the flushing liquid by using the flushing pump assembly and the flushing assembly. Compared with priming the first flushing pipeline, the second flushing pipeline, and the inner cavity of the medical catheter pump with the flushing liquid by using the flushing pump assembly, the flushing assembly is introduced to prime the flushing liquid at the same time, the flushing liquid provided by the flushing assembly is mainly configured to prime the inner cavity of the medical catheter pump, and an additional flushing liquid flow rate is introduced to accelerate an overall flushing process.

Compared with a manner of flushing the flushing path and the inner cavity in a relevant art (in this manner, the flushing liquid passes through the flushing path first and then enters the inner cavity, and the flushing liquid is pumped by the flushing pump assembly, so it is difficult to form a high flow rate, it is difficult to exhaust the air in the inner cavity, and air bubbles will remain in the inner cavity), in this embodiment of the present application, an additional flushing assembly is introduced to prime the inner cavity of the medical catheter pump, so that the flushing liquid can be rapidly injected into the inner cavity, the air in the inner cavity is exhausted more rapidly, and residual air bubbles do not appear easily at a great instantaneous flow rate. Compared with a manner of performing priming by pumping the flushing liquid into the inner cavity by using the flushing pump assembly, and a better exhausting effect for performing priming on the inner cavity is achieved by providing the great instantaneous flow rate by using the rapid flushing assembly.

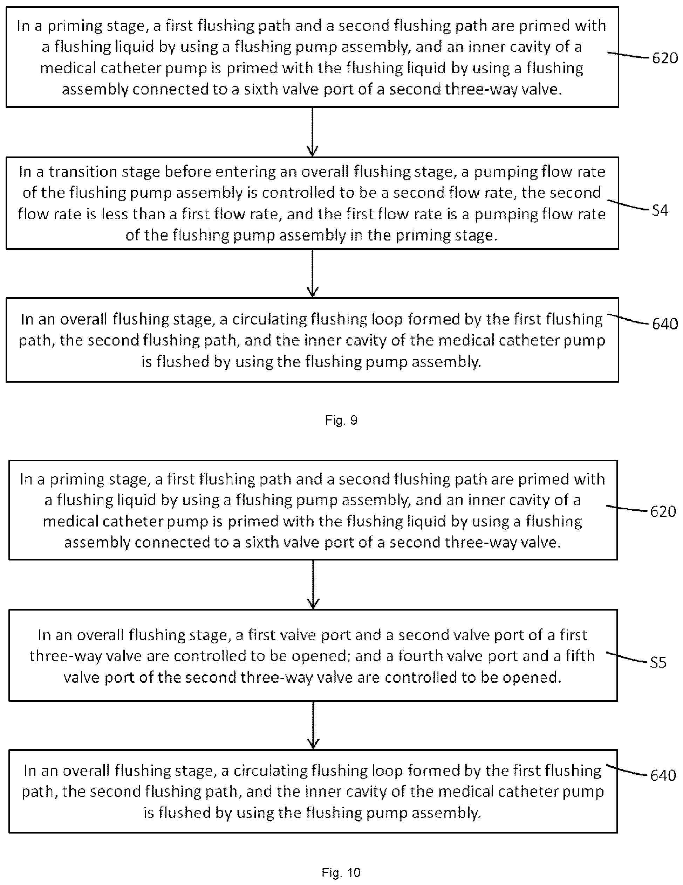

Based on an optional embodiment shown in Fig. 6, Fig. 9 is a flowchart of a flushing method for a medical catheter pump system according to an exemplary embodiment of the present application. S4 is further included before step 640.

At S4, in a transition stage before entering the overall flushing stage, a pumping flow rate of the flushing pump assembly is controlled to be a second flow rate, the second flow rate is less than a first flow rate, and the first flow rate is a pumping flow rate of the flushing pump assembly in the priming stage.

Optionally, in the transition stage before entering the overall flushing stage, a control device controls a pumping flow rate of the flushing pump assembly to be a second flow rate. Optionally, the second flow rate is a flow rate in a catheter of the medical catheter pump. Optionally, the second flow rate does not exceed an acceptable inflow rate of an organism.

In an optional embodiment, in the transition stage, a control device displays prompt information for switching to the overall flushing stage; and a confirm operation is received in response to the prompt information, and the overall flushing stage is entered. For example, the prompt information "please turn three-way valves at a flushing liquid outlet and a flushing liquid inlet of a medical catheter pump to a designated position!", and for example, "overall flushing is about to be performed, please confirm".

In an embodiment, in the transition stage, any two valve ports of the first three-way valve are controlled to communicate with each other, and the fifth valve port and the sixth valve port of the second three-way valve are controlled to communicate with each other.

In another embodiment, in the transition stage, the second valve port and the third valve port of the first three-way valve are controlled to communicate with each other; and the fifth valve port and the sixth valve port of the second three-way valve are controlled to communicate with each other.

In yet another embodiment, in the transition stage, any two valve ports of the second three-way valve are controlled to communicate with each other, and the second valve port and the three valve port of the first three-way valve are controlled to communicate with each other.

The above three conduction states are three conduction states that are formed in the priming stage. During the transition stage, it is possible to simply wait and maintain the conduction states of the three-way valve remaining in the priming stage until the three-way valve is adjusted to a required on-off state, thereby entering the overall flushing stage.

Optionally, the flushing pump assembly includes a flushing pump and a circulating pump. The flushing pump is disposed at a common part of the first flushing path and the second flushing path located at a starting position, and the circulating pump is disposed on a path on the second flushing path that is not shared with the first flushing path.

In an embodiment, a rotating speed of the flushing pump and a rotating speed of the circulating pump are controlled to enable a difference between a flushing liquid flow rate of a first sub-path and a flushing fluid flow rate of a second sub-path to be within a preset range. Optionally, the flushing liquid flow rate of the first sub-path is the same as or similar to the flushing fluid flow rate of the second sub-path, where the first sub-path includes a path on the first flushing path that is not overlapped with the second flushing path, and the second sub-path includes a path on the second flushing path that is not overlapped with the first flushing path; and the first flushing path and the second flushing path are primed with the flushing liquid by using the flushing pump and the circulating pump.

Optionally, the medical catheter pump includes a drive catheter handle, a catheter, and a pump head. A pumping flow rate of the above flushing pump assembly refers to a flushing liquid flow rate in the catheter of the medical catheter pump.

In conclusion, in the transition stage, the pumping flow rate of the flushing pump assembly is reduced, thereby preventing excessive loss of the flushing liquid. Moreover, in the transition stage, the flushing pump assembly only needs to be controlled by the control device to reduce the work, so as to form the described low flushing liquid flow, and wait for the flushing pipeline and the three-way valve to switch to the connection mode and the conduction stage corresponding to the whole flushing stage, thereby reducing the waste of the flushing liquid in the transition stage between the priming stage and the overall flushing stage.

Based on an optional embodiment shown in Fig. 6, Fig. 10 is a flowchart of a flushing method for a medical catheter pump system according to an exemplary embodiment of the present application. S5 is further included before step 640.

At S5, in an overall flushing stage, a first valve port and a second valve port of a first three-way valve are controlled to communicate with each other; and a fourth valve port and a fifth valve port of the second three-way valve are controlled to communicate with each other.

With reference to Fig. 5, Fig. 4 illustrates that a first valve port 221 and a second valve port 222 of the first three-way valve 220 are conducted, and a fourth valve port 231 and a fifth valve port 232 of the second three-way valve 230 are conducted in this case.

Optionally, the flushing pump assembly includes a flushing pump and a circulating pump. The flushing pump is disposed at a common part of the first flushing path and the second flushing path located at a starting position, and the circulating pump is disposed on a path on the second flushing path that is not shared with the first flushing path.

Step 640 includes that: in the overall flushing stage, a rotating speed of the flushing pump is controlled based on a flushing liquid flow rate of a catheter of the medical catheter pump; a rotating speed of the circulating pump is controlled based on a flushing liquid flow rate of the circulating flushing loop formed by the first flushing path, the second flushing path, and the inner cavity of the medical catheter pump; and the circulating flushing loop and the catheter of the medical catheter pump are flushed by using the flushing pump and the circulating pump.

Optionally, the flushing pump and the circulating pump are peristaltic pumps that pump the flushing liquid by squeezing a flushing pipeline.

It is to be noted that, the present application is applied to flush a medical catheter pump. The medical catheter pump is an interventional catheter pump in an organism. The overall flushing stage of the medical catheter pump is performed in the organism. The organism cannot accept an excessively high inflow rate. A rotating speed of the flushing pump enables flushing liquid flow rate in the catheter of the medical catheter pump to be less than an acceptable inflow rate for the organism. A rotating speed of the circulating pump enables the circulating flushing loop to cyclically flush at a high speed.

For example, the flushing pump is controlled to rotate at a first rotating speed to correspondingly implement a flow rate in the catheter of the medical catheter pump as a first target flow rate, the first target flow rate does not exceed the acceptable inflow rate for the organism, and the first target flow rate is equal or similar to a flushing liquid flow rate entering a human body through a pump head. For example, the circulating pump is controlled to rotate at a second rotating speed to correspondingly implement a second target flow rate, the second target flow rate is equal or similar to a sum value of a circulating flow rate in the inner cavity of the medical catheter pump and an inflow rate of the flushing liquid in the catheter of the medical catheter pump, and the second target flow rate is greater than a preset threshold to implement cyclically flushing at a high speed of the flushing liquid in the inner cavity of the medical catheter pump.

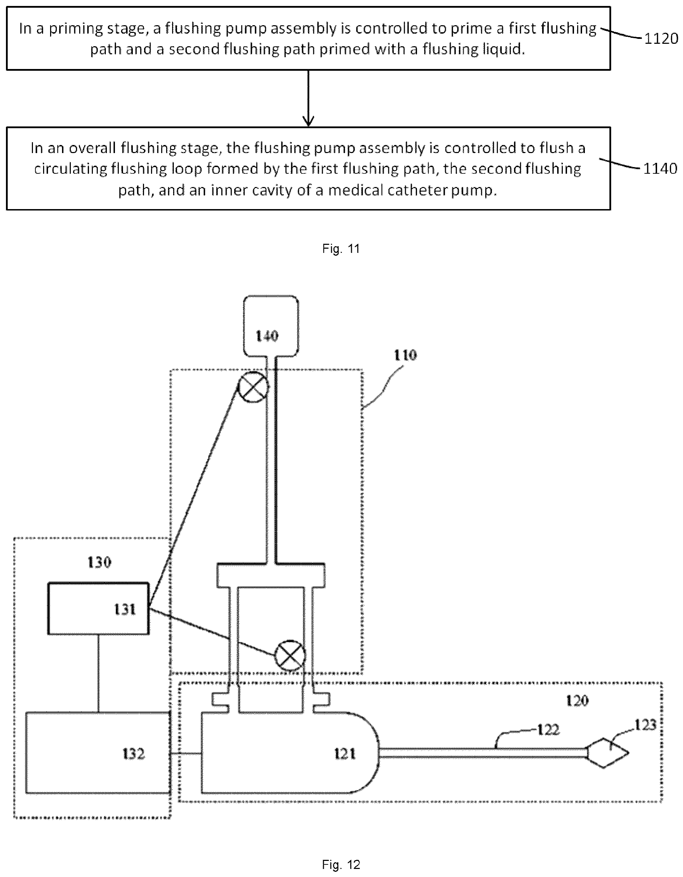

Fig. 11 is a flushing method for a medical catheter pump system according to an exemplary embodiment of the present application. The medical catheter pump system includes a medical catheter pump and at least one flushing pipeline. The method is performed by a control device corresponding to the flushing pump assembly, the control device is connected to the flushing pump assembly. The method includes the following steps.

At step 1120, in a priming stage, a flushing pump assembly is controlled to prime a first flushing path and a second flushing path primed with a flushing liquid.

With reference to Fig. 2, in the present application, a first flushing interface (not shown in the figure) of a medical catheter pump 240 is connected to the first flushing path driven by a flushing pump assembly 210 through an in-valve path between a second valve port 222 and a first valve port 221 of a first three-way valve 220. The first three-way valve 220 further has a third valve port 223. Optionally, the ventricular assist system further includes a flushing liquid container 250. The first flushing path includes a path formed by connecting the first three-way valve 220 and the flushing liquid container 250 by using a flushing pipeline.

A second flushing interface (not shown in the figure) of the medical catheter pump 240 is connected to the second flushing path driven by the flushing pump assembly 210 through an in-valve path between a fifth valve port 232 and a fourth valve port 231 of the second three-way valve 230. The second three-way valve 230 further has a sixth valve port 233. Optionally, the ventricular assist system further includes a flushing liquid container 250. The second flushing path includes a path formed by connecting the second three-way valve 230 and the flushing liquid container 250 by using a flushing pipeline.

At least one flushing pipeline forms the first flushing path and the second flushing path, as described in the embodiment in Fig. 3. Details are not described herein again. Optionally, the flushing pump assembly includes a flushing pump and a circulating pump. The flushing pump is disposed at an overlapped part of the first flushing path and the second flushing path located at a starting position, and the circulating pump is disposed on a path on the second flushing path that is not overlapped with the first flushing path.

In an embodiment, in the priming stage, a rotating speed of the flushing pump and a rotating speed of a circulating pump are controlled to enable a difference between a flushing liquid flow rate of a first sub-path and a flushing fluid flow rate of a second sub-path to be within a preset range, where the first sub-path includes a path on the first flushing path that is not overlapped with the second flushing path, and the second sub-path includes a path on the second flushing path that is not overlapped with the first flushing path; and the first flushing path and the second flushing path are primed with the flushing liquid by using the flushing pump and the circulating pump.

For the rest of content, please refer to relevant introductions in the above Fig. 6 to Fig. 10. Details are not described herein again.

At step 1140, in an overall flushing stage, the flushing pump assembly is controlled to flush a circulating flushing loop formed by the first flushing path, the second flushing path, and an inner cavity of a medical catheter pump.

In an embodiment, a transition stage is further included before the overall flushing stage. In the transition stage before entering the overall flushing stage, a pumping flow rate of the flushing pump is controlled to be a second flow rate, the second flow rate is less than a first flow rate, and the first flow rate is a pumping flow rate of the flushing pump assembly in the priming stage.

In an embodiment, in the overall flushing stage, a rotating speed of the flushing pump is controlled based on a flushing liquid flow rate of a catheter of the medical catheter pump; a rotating speed of the circulating pump is controlled based on a flushing liquid flow rate of the circulating flushing loop formed by the first flushing path, the second flushing path, and the inner cavity of the medical catheter pump; and the circulating flushing loop and the catheter of the medical catheter pump are flushed by using the flushing pump and the circulating pump.

For the rest of content, please refer to relevant introductions in the above Fig. 6 to Fig. 10. Details are not described herein again.

In conclusion, the first three-way valve and the second three-way valve have different conduction states when flushing or priming is performed on different flushing positions. The flushing positions include, but are not limited to, the first flushing path, the second flushing path, or the inner cavity of the medical catheter pump. Different positions in the ventricular assist system can be separately flushed or primed by switching the conduction state of the first three-way valve and the second three-way valve to implement priming or flushing for different flushing positions separately. Compared with priming or flushing the overall position at one time, the method for dividing the flushing positions to perform priming or flushing can enable each flushing position to be sufficiently flushed or primed, thereby exhausting air at different flushing positions more thoroughly, avoiding air bubbles remaining in some narrow gaps, and improving an exhaust effect of priming or flushing an overall system.

Moreover, the first three-way valve and the second three-way valve are disposed between the flushing path and the medical catheter pump, so that the medical catheter pump can be connected to an additional flushing assembly through the three-way valves, and the medical catheter pump is flushed by using the additional flushing assembly, thereby exhausting the air in the inner cavity of the medical catheter pump, and preventing air bubbles from remaining in small gaps in the inner cavity of the medical catheter pump.

The present application provides a consumable material for a medical catheter pump system. The consumable material for the medical catheter pump includes a first three-way valve and a second three-way valve.

A first valve port of the first three-way valve is connected to a first flushing path driven by a flushing pump assembly, and a second valve port of the first three-way valve is connected to a first flushing interface of a medical catheter pump.

A fourth valve port of the second three-way valve is connected to a second flushing path driven by the flushing pump assembly, and a fifth valve port of the second three-way valve is connected to a second flushing interface of the medical catheter pump.

In an embodiment, the combustible material for the medical catheter pump system further includes a flushing pipeline, wherein the flushing pipeline forms the first flushing path and the second flushing path, the first three-way valve is disposed at a tail end of a pipe orifice corresponding to the first flushing path, and the second three-way valve is disposed at a tail end of a pipe orifice corresponding to the second flushing path.

In an embodiment, the medical catheter pump system further includes a medical catheter pump. The medical catheter pump includes: a catheter, a drive shaft, a pump head, and a drive catheter handle.

The drive shaft rotatably penetrates through the catheter.

The pump head includes: a pump housing connected to a distal end of the catheter, and an impeller accommodated in the pump housing, wherein the impeller is connected to a distal end of the drive shaft to be driven to rotate to pump blood.

The drive catheter handle includes a rotor capable of being driven by a motor, wherein a proximal end of the drive shaft is connected to the rotor, and an inner cavity for rotatably supporting the rotor in the drive catheter handle is formed in the drive catheter handle; the catheter is communicated with the inner cavity; and the drive catheter handle is provided with the first flushing interface and the second flushing interface that are communicated with the inner cavity, the first three-way valve is disposed at the first flushing interface, and the second three-way valve is disposed at the second flushing interface.

Part of a flushing liquid entering the inner cavity through the second flushing interface enters the catheter and is completely discharged to a human body at the pump head, and the other part is discharged from the first flushing interface after passing through the rotor.

Fig. 12 is a schematic diagram of a ventricular assist system according to another exemplary embodiment of the present application. The ventricular assist system is a transcatheter ventricular assist system, including a control device, and a medical catheter pump system matched with the control device. The control device 130 at least includes a console 131 and a drive motor 132. Optionally, the control device 130 further includes a flushing pump assembly (not shown in the figure), and the flushing pump assembly includes a flushing pump and a circulating pump. Clinical medical staff can monitor a system state and patient physiological parameters on a control host interface, and provide varying degrees of circulation assistance by adjusting a rotating speed of a catheter pump according to patient needs, thereby temporarily maintaining blood circulation of important organs of a patient and unloading a load on the heart.

A catheter pump system includes a catheter pump 120, a delivery system (not shown in the figure), and a flushing pipeline 110.

The catheter pump 120 includes a pump head 123 (containing an impeller, a bracket, and a membrane), a catheter 122, a drive catheter handle 121 (containing a drive catheter locking connecting piece), a flushing pressure sensor, and a drive catheter flushing pipeline connector. The catheter pump can be percutaneously placed into the heart through a peripheral blood vessel, and the pump head 123 is placed between the left ventricle and the ascending aorta. A blood inlet of the pump head 123 is placed into the left ventricle, a blood outlet of the pump head 123 is placed into the ascending aorta, the drive motor 132 is connected to an interventional pump to drive the impeller in the pump head 123 to rotate, thereby pumping blood from the left ventricle into the ascending aorta, and implementing a ventricular assist function.

The delivery system includes a dilator, an interventional sheath, an introducer. The delivery system is configured to dilate a blood vessel and provide a path for placing the interventional pump into the heart. The introducer and the interventional sheath have a function of folding the pump head. The introducer and the dilator will be removed after the interventional is placed in place. The interventional sheath will continuously exit in a patient vessel until the interventional pump is removed. When the interventional pump is removed, the interventional sheath also achieves an effect of folding the pump head.

The flushing pipeline 110 at least includes the above supplementary pipeline, communication structure, flushing liquid inlet pipe, and flushing liquid outlet pipe.

The flushing pump is connected to the flushing pipeline 110. The flushing pump implements flushing liquid delivery and control functions by squeezing the flushing pipeline, thereby preventing blood from entering a drive catheter of the interventional pump to generate a thrombus.

The flushing pump drives the flushing pipeline to flush a flushing pump pipe in a kit, and a flushing liquid in a liquid bag 140 connected to the flushing pipe can be pumped into a cavity of the interventional pump through the flushing pipe, thereby preventing the blood from entering the catheter 122 of the interventional pump.

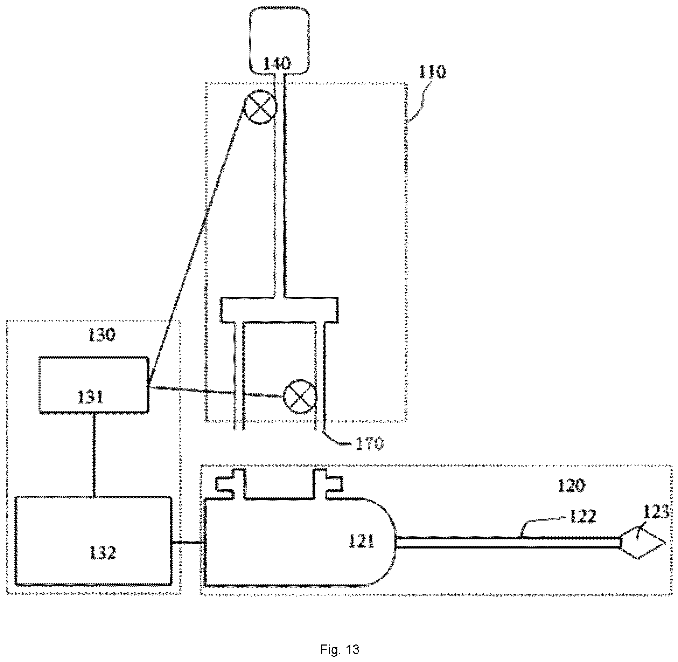

In an embodiment, a three-way valve belongs to a consumable material in the catheter pump 120. For example, with reference to Fig. 13, Fig. 13 illustrates that a three-way valve belongs to a consumable material in a catheter pump. In this case, the flushing pipeline has a joint 170 configured to connect the three-way valve. Optionally, the joint 170 may be a Luer taper.

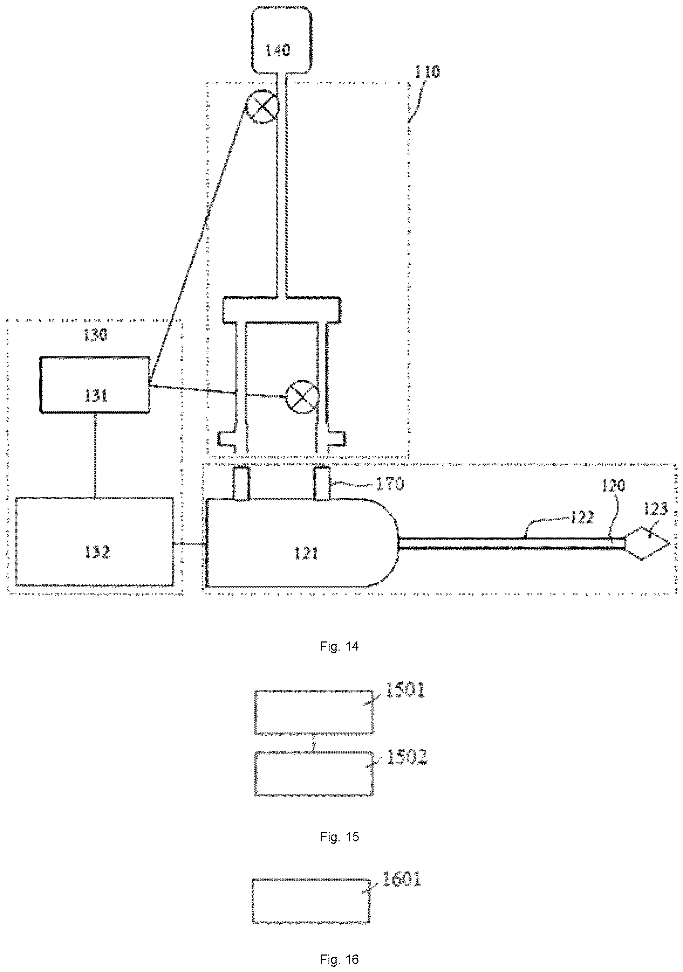

In an embodiment, a three-way valve belongs to a consumable material in the flushing pipeline 110. For example, with reference to Fig. 14, Fig. 14 illustrates that a three-way valve belongs a consumable material in a flushing pipeline. In this case, the drive catheter handle 121 has a joint 170 configured to connect the three-way valve. Optionally, the joint 170 is a Luer taper.

Fig. 15 is a control apparatus for a medical catheter pump system according to an exemplary embodiment of the present application. The medical catheter pump system includes a medical catheter pump and a flushing pipeline; a first flushing interface of the medical catheter pump is connected to a first flushing path driven by a flushing pump assembly through an in-valve path between a second valve port and a first valve port of a first three-way valve; a second flushing interface of the medical catheter pump is connected to a second flushing path driven by the flushing pump assembly through an in-valve path between a fifth valve port and a fourth valve port of a second three-way valve; the flushing pipeline forms the first flushing path and the second flushing path. The apparatus includes a control module 1501.

The control module 1501 is configured to: in a priming stage, prime the first flushing path and the second flushing path with a flushing liquid by using the flushing pump assembly, and prime an inner cavity of the medical catheter pump with the flushing liquid by using a flushing assembly connected to a sixth valve port of the second three-way valve.

The control module 1501 is further configured to: in an overall flushing stage, flush a circulating flushing loop formed by the first flushing path, the second flushing path, and the inner cavity of the medical catheter pump by using the flushing pump assembly.

In an optional embodiment, the control module 1501 is further configured to: in a first priming stage, prime the inner cavity of the medical catheter pump with the flushing liquid by using the flushing assembly connected to a sixth valve port of the second three-way valve; and in a second priming stage, the first flushing path and the second flushing path are primed with the flushing liquid by using the flushing pump assembly.

In an optional embodiment, the control module 1501 is further configured to: in the first priming stage, control the second valve port and the third valve port of the first three-way valve to be conducted; and control the fifth valve port and the sixth valve port of the second three-way valve to be conducted.

In an optional embodiment, the control module 1501 is further configured to: in the second priming stage, control the first valve port and the third valve port of the first three-way valve to be conducted; and control the fourth valve port and the sixth valve port of the second three-way valve to be conducted.

In an optional embodiment, the control module 1501 is further configured to: in the priming stage, prime the inner cavity of the medical catheter pump with the flushing liquid by using the flushing assembly connected to the sixth valve port of the second three-way valve; and simultaneously, prime the first flushing path and the second flushing path with the flushing liquid by using the flushing pump assembly.

In an optional embodiment, the control module 1501 is further configured to: in the priming stage, control any two valve ports of the first three-way valve to be conducted; and control the fifth valve port and the sixth valve port of the second three-way valve to be conducted.

In an optional embodiment, the control module 1501 is further configured to: in the priming stage, control the second valve port and the third valve port of the first three-way valve to be conducted; and control the fifth valve port and the sixth valve port of the second three-way valve to be conducted.

In an optional embodiment, the flushing assembly includes a rapid flushing assembly. The control module 1501 is further configured to prime the inner cavity of the medical catheter pump with the flushing liquid by using the rapid flushing assembly connected to the sixth valve port of the second three-way valve, and an instantaneous flow rate of the flushing liquid provided by the rapid flushing assembly is greater than an instantaneous flow rate of the flushing liquid provided by the flushing pump assembly.

In an optional embodiment, the flushing pump assembly includes a flushing pump and a circulating pump. The flushing pump is disposed at a common part of the first flushing path and the second flushing path located at a starting position, and the circulating pump is disposed on a path on the second flushing path that is not shared with the first flushing path. The control module 1501 is further configured to: control a rotating speed of the flushing pump and a rotating speed of a circulating pump to enable a difference between a flushing liquid flow rate of a first sub-path and a flushing fluid flow rate of a second sub-path to be within a preset range, where the first sub-path includes a path on the first flushing path that is not overlapped with the second flushing path, and the second sub-path includes a path on the second flushing path that is not overlapped with the first flushing path; and prime the first flushing path and the second flushing path with the flushing liquid by using the flushing pump and the circulating pump.

In an optional embodiment, the control module 1501 is further configured to: in a transition stage before entering the overall flushing stage, control a pumping flow rate of the flushing pump assembly to be a second flow rate, the second flow rate is less than a first flow rate, and the first flow rate is a pumping flow rate of the flushing pump assembly in the priming stage.

In an optional embodiment, the control module 1501 is further configured to: in the transition stage, control any two valve ports of the first three-way valve to be conducted; and control the fifth valve port and the sixth valve port of the second three-way valve to be conducted.

In an optional embodiment, the control module 1501 is further configured to: in the transition stage, control the second valve port and the third valve port of the first three-way valve to be conducted; and control the fifth valve port and the sixth valve port of the second three-way valve to be conducted.

In an optional embodiment, the apparatus further includes a display module 1502. The display module 1502 is configured to: in the transition stage, display prompt information for switching to the overall flushing stage; and receive a confirm operation in response to the prompt information, and enter the overall flushing stage.

In an optional embodiment, the control module 1501 is further configured to: in the overall flushing stage, control the first valve port and the second valve port of the first three-way valve to be conducted; and control the fourth valve port and the fifth valve port of the second three-way valve to be conducted.

In an optional embodiment, the flushing pump assembly includes a flushing pump and a circulating pump. The flushing pump is disposed at a common part of the first flushing path and the second flushing path located at a starting position, and the circulating pump is disposed on a path on the second flushing path that is not shared with the first flushing path. The control module 1501 is further configured to: in the overall flushing stage, control a rotating speed of the flushing pump based on a flushing liquid flow rate of a catheter of the medical catheter pump; control a rotating speed of the circulating pump based on a flushing liquid flow rate of the circulating flushing loop formed by the first flushing path, the second flushing path, and the inner cavity of the medical catheter pump; and flush the circulating flushing loop and the catheter of the medical catheter pump by using the flushing pump and the circulating pump.

In conclusion, the first three-way valve and the second three-way valve are disposed between the flushing pump assembly and the medical catheter pump, so that the medical catheter pump can be connected to an additional flushing assembly through the three-way valves, and the medical catheter pump is flushed by using the additional flushing assembly, thereby eliminating air bubbles in small gaps in the medical catheter pump.

Moreover, the additional flushing assembly reduces a pump speed pressure of the flushing pump assembly, the flushing pump assembly is not configured to prime all flushing positions with a flushing liquid, but is only configured to prime part flushing positions with the flushing liquid.

Fig. 16 is a control apparatus for a medical catheter pump system according to an exemplary embodiment of the present application. The medical catheter pump system includes a medical catheter pump and a flushing pipeline; a first flushing interface of the medical catheter pump is connected to a first flushing path driven by a flushing pump assembly through an in-valve path between a second valve port and a first valve port of a first three-way valve; a second flushing interface of the medical catheter pump is connected to a second flushing path driven by the flushing pump assembly through an in-valve path between a fifth valve port and a fourth valve port of a second three-way valve; the flushing pipeline forms the first flushing path and the second flushing path. The apparatus includes a control module 1601.

The control module 1601 configured to: in a priming stage, control the flushing pump assembly to prime the first flushing path and the second flushing path with a flushing liquid; and
in an overall flushing stage, control the flushing pump assembly to flush a circulating flushing loop formed by the first flushing path, the second flushing path, and the inner cavity of the medical catheter pump.

In an optional embodiment, the flushing pump assembly includes a flushing pump and a circulating pump. The flushing pump is disposed at a common part of the first flushing path and the second flushing path located at a starting position, and the circulating pump is disposed on a path on the second flushing path that is not shared with the first flushing path. The control module 1601 is further configured to: in a priming stage, control a rotating speed of the flushing pump and a rotating speed of a circulating pump to enable a difference between a flushing liquid flow rate of a first sub-path and a flushing fluid flow rate of a second sub-path to be within a preset range, where the first sub-path includes a path on the first flushing path that is not overlapped with the second flushing path, and the second sub-path includes a path on the second flushing path that is not overlapped with the first flushing path; and prime the first flushing path and the second flushing path with the flushing liquid by using the flushing pump and the circulating pump.

In an optional embodiment, the control module 1601 is further configured to: control a pumping flow rate of the flushing pump to be a second flow rate, the second flow rate is less than a first flow rate, and the first flow rate is a pumping flow rate of the flushing pump assembly in the priming stage.

In an optional embodiment, the flushing pump assembly includes a flushing pump and a circulating pump. The flushing pump is disposed at a common part of the first flushing path and the second flushing path located at a starting position, and the circulating pump is disposed on a path on the second flushing path that is not shared with the first flushing path. The control module 1601 is further configured to: in the overall flushing stage, control a rotating speed of the flushing pump based on a flushing liquid flow rate of a catheter of the medical catheter pump; control a rotating speed of the circulating pump based on a flushing liquid flow rate of the circulating flushing loop formed by the first flushing path, the second flushing path, and the inner cavity of the medical catheter pump; and flush the circulating flushing loop and the catheter of the medical catheter pump by using the flushing pump and the circulating pump.

In an optional embodiment, the control apparatus further includes a flushing pump assembly. The flushing pump assembly includes a flushing pump and a circulating pump. The flushing pump is configured to deliver the flushing liquid into the inner cavity of the medical catheter pump through the second flushing path in the overall flushing stage, and the circulating pump is configured to control the flushing liquid to cyclically flush the inner cavity of the medical catheter pump in the overall flushing stage.

In an optional embodiment, the flushing pump is disposed at a common part of the first flushing path and the second flushing path located at a starting position, and the circulating pump is disposed on a path on the second flushing path that is not shared with the first flushing path.

In conclusion, the first three-way valve and the second three-way valve are disposed between the flushing path and the medical catheter pump, so that the medical catheter pump can be connected to an additional flushing assembly through the three-way valves, and the medical catheter pump is flushed by using the additional flushing assembly, thereby eliminating air bubbles in small gaps in the medical catheter pump.

Moreover, the additional flushing assembly reduces a pump speed pressure of the flushing pump assembly, the flushing pump assembly is not configured to prime all flushing positions with a flushing liquid, but is only configured to prime part flushing positions with the flushing liquid.

Embodiments of the present application further provide a control device. The control device is configured to implement the method for flushing the medical catheter pump system according to the above method embodiments.

Embodiments of the present application further provide a computer storage medium. The computer storage medium stores at least one computer program. The at least one computer program is loaded and executed by a processor to implement the method for flushing the medical catheter pump system according to the above method embodiments.

Embodiments of the present application further provide a chip. The chip includes a programmable logic circuit or a program, and a device installed with the chip is configured to implement the method for flushing the medical catheter pump system as described in the above aspect.

Embodiments of the present application further provide a computer program product. The computer program product includes a computer program, and the computer program is stored in a computer storage medium. The computer program is read and executed by a processor of the computer device from the computer storage medium, so that the computer device performs the method for flushing the medical catheter pump system according to the above method embodiments.

It is to be noted that, unless otherwise explicitly defined herein, all terms used in the claims are interpreted according to ordinary meanings in the technical field. Unless otherwise explicitly stated, all references to "an member, apparatus, component, device, step, and the like" are openly interpreted as referring to at least one example of the member, apparatus, component, device, step, and the like. Unless explicitly stated, steps of any method disclosed herein do not have to be performed in an exact sequence disclosed.

It is to be understood that "a plurality of" mentioned herein refers to two or more. "And/or" describes an associative relationship between associated objects, indicating that three relationships can exist. For example, A and/or B can indicate: presence of A alone, presence of both A and B, and presence of B alone. The character "/" generally indicates an "or" relationship between associated objects before and after.

Those of ordinary skill in the art can understand that all or part steps of the above embodiments are implemented by hardware, or are implemented by a program instructing relevant hardware. The program is stored in a computer storage medium. The storage medium mentioned above is a read-only memory, a magnetic disk, an optical disc, or the like.

The above descriptions are merely optional embodiments of the present application, but are not intended to limit the present application. Any modification, equivalent switching, or improvement made within the spirit and principle of the present application fall within the scope of protection of the present application.

## Claims

1. A ventricular assist system, comprising a flushing pump assembly, a first three-way valve, a second three-way valve, and a medical catheter pump, wherein
the first three-way valve comprises a first valve port, a second valve port, and a third valve port, the first valve port is connected to a first flushing path driven by the flushing pump assembly, and the second valve port is connected to a first flushing interface of the medical catheter pump; and
the second three-way valve comprises a fourth valve port, a fifth valve port, and a sixth valve port, the fourth valve port is connected to a second flushing path driven by the flushing pump assembly, and the fifth valve port is connected to a second flushing interface of the medical catheter pump.

2. The ventricular assist system according to claim 1, wherein the first three-way valve and the second three-way valve have different conduction states when flushing or priming for different flushing positions are different, the flushing positions comprise the first flushing path and the second flushing path, or the flushing positions comprise an inner cavity of the medical catheter pump.

3. A flushing method for a medical catheter pump system, wherein the medical catheter pump system comprises a medical catheter pump and a flushing pipeline; a first flushing interface of the medical catheter pump is connected to a first flushing path driven by a flushing pump assembly through an in-valve path between a second valve port and a first valve port of a first three-way valve; a second flushing interface of the medical catheter pump is connected to a second flushing path driven by the flushing pump assembly through an in-valve path between a fifth valve port and a fourth valve port of a second three-way valve; the flushing pipeline forms the first flushing path and the second flushing path; and
the method comprises: in a priming stage, priming the first flushing path and the second flushing path with a flushing liquid by using the flushing pump assembly, and priming an inner cavity of the medical catheter pump with the flushing liquid by using a flushing assembly connected to a sixth valve port of the second three-way valve or a third valve port of the first three-way valve; and
in an overall flushing stage, flushing a circulating flushing loop formed by the first flushing path, the second flushing path, and the inner cavity of the medical catheter pump by using the flushing pump assembly.

4. The method according to claim 3, wherein the in a priming stage, priming the first flushing path and the second flushing path with a flushing liquid by using the flushing pump assembly, and priming an inner cavity of the medical catheter pump with the flushing liquid by using a flushing assembly connected to a sixth valve port of the second three-way valve or a third valve port of the first three-way valve comprises:
in a first priming stage, priming the inner cavity of the medical catheter pump with the flushing liquid by using the flushing assembly connected to the sixth valve port of the second three-way valve or the third valve port of the first three-way valve; and
in a second priming stage, priming the first flushing path and the second flushing path with the flushing liquid by using the flushing pump assembly.

5. The method according to claim 4, wherein
in the first priming stage, the second valve port and the third valve port of the first three-way valve are controlled to communicate with each other, and the fifth valve port and the sixth valve port of the second three-way valve are controlled to communicate with each other; and
in the second priming stage, the first valve port and the third valve port of the first three-way valve are controlled to communicate with each other, and the fourth valve port and the sixth valve port of the second three-way valve are controlled to communicate with each other.

6. The method according to claim 3, wherein
in the priming stage, any two valve ports of the first three-way valve are controlled to communicate with each other, and the fifth valve port and the sixth valve port of the second three-way valve are controlled to communicate with each other; or
in the priming stage, any two valve ports of the second three-way valve are controlled to communicate with each other, and the second valve port and the third valve port of the first three-way valve are controlled to communicate with each other; or
the flushing assembly comprises a rapid flushing assembly; the priming an inner cavity of the medical catheter pump with the flushing liquid by using a flushing assembly connected to a sixth valve port of the second three-way valve or a third valve port of the first three-way valve comprises:
priming the inner cavity of the medical catheter pump with the flushing liquid by using the rapid flushing assembly connected to the sixth valve port of the second three-way valve or the third valve port of the first three-way valve, wherein an instantaneous flow rate of the flushing liquid provided by the rapid flushing assembly is greater than an instantaneous flow rate of the flushing liquid provided by the flushing pump assembly.

7. The method according to claim 3, further comprising:
in the overall flushing stage, the first valve port and the second valve port of the first three-way valve are controlled to communicate with each other, and the fourth valve port and the fifth valve port of the second three-way valve are controlled to communicate with each other.

8. The method according to claim 3, wherein the flushing pump assembly comprises a flushing pump and a circulating pump, the flushing pump is disposed at a common part of the first flushing path and the second flushing path located at a starting position, and the circulating pump is disposed on a path on the second flushing path that is not shared with the first flushing path, wherein,
the priming the first flushing path and the second flushing path with a flushing liquid by using the flushing pump assembly comprises:
controlling a rotating speed of the flushing pump and a rotating speed of the circulating pump to enable a difference between a flushing liquid flow rate of a first sub-path and a flushing fluid flow rate of a second sub-path to be within a preset range, wherein the first sub-path comprises a path on the first flushing path that is not shared with the second flushing path, and the second sub-path comprises a path on the second flushing path that is not shared with the first flushing path;
priming the first flushing path and the second flushing path with the flushing liquid by using the flushing pump and the circulating pump; or
the in an overall flushing stage, flushing a circulating flushing loop formed by the first flushing path, the second flushing path, and the inner cavity of the medical catheter pump by using the flushing pump assembly comprises:
in the overall flushing stage, controlling a rotating speed of the flushing pump based on a flushing liquid flow rate of a catheter of the medical catheter pump;
and controlling a rotating speed of the circulating pump based on a flushing liquid flow rate of the circulating flushing loop formed by the first flushing path, the second flushing path, and the inner cavity of the medical catheter pump; and
flushing the circulating flushing loop and the catheter of the medical catheter pump by using the flushing pump and the circulating pump.

9. The method according to claim 3, further comprising:
in a transition stage before entering the overall flushing stage, controlling a pumping flow rate of the flushing pump assembly to be a second flow rate, wherein the second flow rate is less than a first flow rate, and the first flow rate is a pumping flow rate of the flushing pump assembly in the priming stage.

10. The method according to claim 9, wherein in the transition stage,
any two valve ports of the first three-way valve are controlled to communicate with each other, and the fifth valve port and the sixth valve port of the second three-way valve are controlled to communicate with each other; or
any two valve ports of the second three-way valve are controlled to communicate with each other, and the second valve port and the three valve port of the first three-way valve are controlled to communicate with each other.

11. The method according to claim 10, further comprising:
in the transition stage, displaying prompt information for switching to the overall flushing stage; and
receiving a confirm operation in response to the prompt information, and entering the overall flushing stage.

12. A flushing method for a medical catheter pump system, wherein the medical catheter pump system comprises a medical catheter pump and a flushing pipeline; a first flushing interface of the medical catheter pump is connected to a first flushing path driven by a flushing pump assembly through an in-valve path between a second valve port and a first valve port of a first three-way valve; a second flushing interface of the medical catheter pump is connected to a second flushing path driven by the flushing pump assembly through an in-valve path between a fifth valve port and a fourth valve port of a second three-way valve; the flushing pipeline forms the first flushing path and the second flushing path; and
the method is performed by a control device corresponding to the flushing pump assembly, the control device is connected to the flushing pump assembly, and the method comprises:
in a priming stage, controlling the flushing pump assembly to prime the first flushing path and the second flushing path with a flushing liquid; and
in an overall flushing stage, controlling the flushing pump assembly to flush a circulating flushing loop formed by the first flushing path, the second flushing path, and the inner cavity of the medical catheter pump.

13. A consumable material for a medical catheter pump system, comprising a first three-way valve and a second three-way valve;
a first valve port of the first three-way valve is connected to a first flushing path driven by a flushing pump assembly, and a second valve port of the first three-way valve is connected to a first flushing interface of a medical catheter pump; and
a fourth valve port of the second three-way valve is connected to a second flushing path driven by the flushing pump assembly, and a fifth valve port of the second three-way valve is connected to a second flushing interface of the medical catheter pump.

14. The consumable material according to claim 13, further comprising:
a flushing pipeline, wherein the flushing pipeline forms the first flushing path and the second flushing path, the first three-way valve is disposed at a tail end of a pipe orifice corresponding to the first flushing path, and the second three-way valve is disposed at a tail end of a pipe orifice corresponding to the second flushing path; or
the medical catheter pump, wherein the medical catheter pump comprises:
a catheter;
a drive shaft, rotatably penetrating through the catheter;
a pump head, comprising: a pump housing connected to a distal end of the catheter, and an impeller accommodated in the pump housing, wherein the impeller is connected to a distal end of the drive shaft to be driven to rotate to pump blood; and
a drive catheter handle, comprising a rotor capable of being driven by a motor, wherein a proximal end of the drive shaft is connected to the rotor, and an inner cavity for rotatably supporting the rotor in the drive catheter handle is formed in the drive catheter handle; the catheter is communicated with the inner cavity; and the drive catheter handle is provided with the first flushing interface and the second flushing interface that are communicated with the inner cavity, the first three-way valve is disposed at the first flushing interface, and the second three-way valve is disposed at the second flushing interface, wherein
a part of a flushing liquid entering the inner cavity through the second flushing interface enters the catheter and is completely discharged to a human body at the pump head, and an other part is discharged from the first flushing interface after passing through the rotor.

15. A control apparatus for a medical catheter pump system, wherein the medical catheter pump system comprises a medical catheter pump and a flushing pipeline; a first flushing interface of the medical catheter pump is connected to a first flushing path driven by a flushing pump assembly through an in-valve path between a second valve port and a first valve port of a first three-way valve; a second flushing interface of the medical catheter pump is connected to a second flushing path driven by the flushing pump assembly through an in-valve path between a fifth valve port and a fourth valve port of a second three-way valve; the flushing pipeline forms the first flushing path and the second flushing path; and
the apparatus comprises:
a control module, configured to: in a priming stage, prime the first flushing path and the second flushing path with a flushing liquid by using the flushing pump assembly, and prime an inner cavity of the medical catheter pump with the flushing liquid by using a flushing assembly connected to a sixth valve port of the second three-way valve, wherein
the control module is further configured to: in an overall flushing stage, flush a circulating flushing loop formed by the first flushing path, the second flushing path, and the inner cavity of the medical catheter pump by using the flushing pump assembly.

16. A control apparatus for a medical catheter pump system, wherein the medical catheter pump system comprises a medical catheter pump and a flushing pipeline; a first flushing interface of the medical catheter pump is connected to a first flushing path driven by a flushing pump assembly through an in-valve path between a second valve port and a first valve port of a first three-way valve; a second flushing interface of the medical catheter pump is connected to a second flushing path driven by the flushing pump assembly through an in-valve path between a fifth valve port and a fourth valve port of a second three-way valve; the flushing pipeline forms the first flushing path and the second flushing path; and
the control apparatus comprises:
a control module, configured to: in a priming stage, control the flushing pump assembly to prime the first flushing path and the second flushing path with a flushing liquid, wherein
the control module is further configured to: in an overall flushing stage, control the flushing pump assembly to flush a circulating flushing loop formed by the first flushing path, the second flushing path, and the inner cavity of the medical catheter pump.

17. The control apparatus according to claim 16, wherein the flushing pump assembly comprises a flushing pump and a circulating pump, wherein
the flushing pump is disposed at a common part of the first flushing path and the second flushing path located at a starting position, and the flushing pump is configured to deliver the flushing liquid into the inner cavity of the medical catheter pump through the second flushing path in the overall flushing stage; and
the circulating pump is disposed on a path on the second flushing path that is not shared with the first flushing path, and the circulating pump is configured to control the flushing liquid to cyclically flush the inner cavity of the medical catheter pump in the overall flushing stage.

18. A control device, being configured to implement the method for flushing the medical catheter pump system according to any one of claims 3 to 12.

19. A computer storage medium, having at least one computer program stored therein, wherein the at least one computer program is loaded and executed by a processor to implement the method for flushing the medical catheter pump system according to any one of claims 3 to 12.

20. A chip, comprising a programmable logic circuit or a program, wherein a device installed with the chip is configured to implement the method for flushing the medical catheter pump system according to any one of claims 3 to 12.
